# EUROPEAN PATENT SPECIFICATION

(11) **EP 2 601 297 B1**
(45) Date of publication and mention of the grant of the patent: **16.09.2020**
(21) Application number: 11794229.2
(22) Date of filing: 01.08.2011
(51) Int. Cl.: C12N 15/82, C12N 9/22, A01H 13/00

(54) **METHOD FOR TARGETED GENOMIC EVENTS IN ALGAE**
VERFAHREN FÜR GEZIELTE GENOMISCHE EREIGNISSE BEI ALGEN
PROCÉDÉ DESTINÉ À DES ÉVÉNEMENTS GÉNOMIQUES CIBLÉS DANS DES ALGUES

(30) Priority: 02.08.2010 US 370017 P
(43) Date of publication of application: 12.06.2013
(73) Proprietor: Cellectis, 75013 Paris (FR)
(72) Inventor: SOURDIVE, David, F-92300 Levallois-perret (FR)
(74) Representative: Santarelli
(86) International application number: PCT/IB2011/002605
(87) International publication number: WO 2012/017329

(56) References cited:
- WO-A1-2011/104382
- WO-A2-2007/047859
- CN-A- 101 423 830
- US-A- 5 420 032
- SELIGMAN L M ET AL: "Mutations altering the cleavage specificity of a homing endonuclease", NUCLEIC ACIDS RESEARCH, OXFORD UNIVERSITY PRESS, SURREY, GB, vol. 30, no. 17, 1 September 2002 (2002-09-01), pages 3870-3879, XP002282592, ISSN: 0305-1048, DOI: 10.1093/NAR/GKF495
- ARNOULD S ET AL: "Engineering of Large Numbers of Highly Specific Homing Endonucleases that Induce Recombination on Novel DNA Targets", JOURNAL OF MOLECULAR BIOLOGY, ACADEMIC PRESS, UNITED KINGDOM, vol. 355, no. 3, 20 January 2006 (2006-01-20), pages 443-458, XP024950505, ISSN: 0022-2836, DOI: 10.1016/J.JMB.2005.10.065 [retrieved on 2006-01-20]
- LI Y ET AL: "Chlamydomonas starchless mutant defective in ADP-glucose pyrophosphorylase hyper-accumulates triacylglycerol", METABOLIC ENGINEERING, ACADEMIC PRESS, US, vol. 12, no. 4, 1 July 2010 (2010-07-01), pages 387-391, XP027079426, ISSN: 1096-7176, DOI: 10.1016/J.YMBEN.2010.02.002 [retrieved on 2010-02-19] cited in the application
- ZORIN B ET AL: "NUCLEAR-GENE TARGETING BY USING SINGLE-STRANDED DNA AVOIDS ILLEGITIMATE DNA INTEGRATION IN CHLAMYDOMONAS REINHARDTII", EUKARYOTIC CELL, AMERICAN SOCIETY FOR MICROBIOLOGY, US, vol. 4, no. 7, 1 July 2005 (2005-07-01), pages 1264-1272, XP009053638, ISSN: 1535-9778, DOI: 10.1128/EC.4.7.1264-1272.2005
- BATEMAN J M ET AL: "Tools for chloroplast transformation in Chlamydomonas: Expression vectors and a new dominant selectable marker", MOLECULAR AND GENERAL GENETICS, vol. 263, no. 3, April 2000 (2000-04), pages 404-410, XP002671608, ISSN: 0026-8925
- SLANINOVA MIROSLAVA ET AL: "Is it possible to improve homologous recombination in Chlamydomonas reinhardtii?", BIOLOGIA (BRATISLAVA), vol. 63, no. 6, December 2008 (2008-12), pages 941-946, ISSN: 0006-3088(print)
- DUERRENBERGER F ET AL: "Double strand break-induced recombination in Chlamydomonas reinhardtii chloroplasts", NUCLEIC ACIDS RESEARCH, INFORMATION RETRIEVAL LTD, GB, vol. 24, no. 17, 1 January 1996 (1996-01-01), pages 3323-3331, XP002245662, ISSN: 0305-1048, DOI: 10.1093/NAR/24.17.3323
- ODOM OBED W ET AL: "Chlamydomonas chloroplasts can use short dispersed repeats and multiple pathways to repair a double-strand break in the genome.", THE PLANT JOURNAL : FOR CELL AND MOLECULAR BIOLOGY MAR 2008, vol. 53, no. 5, March 2008 (2008-03), pages 842-853, ISSN: 1365-313X
- DE RISO VALENTINA ET AL: "Gene silencing in the marine diatom Phaeodactylum tricornutum", NUCLEIC ACIDS RESEARCH, vol. 37, no. 14, August 2009 (2009-08), page Article No.: e96, ISSN: 0305-1048
- Materna C A: "Development of molecular tools in thediatom Phaeodactylum tricornutum", Dissertation, Chapter II.2 , 21 December 2006 (2006-12-21), pages 41-63, Retrieved from the Internet: URL:https://pdfs.semanticscholar.org/81b5/ 66151fb99e0efe70a8eb023ee318a4d866c7.pdf
- DE RISO VALENTINA ET AL: "Gene silencing in the marine diatom Phaeodactylum tricornutum", NUCLEIC ACIDS RESEARCH, vol. 37, no. 14, August 2009 (2009-08), page Article No.: e96, ISSN: 0305-1048
- Michael H. Huesemann ET AL: "Biomass Productivities in Wild Type and Pigment Mutant of Cyclotella sp. (Diatom)", Applied Biochemistry and Biotechnology, vol. 157, no. 3, 3 July 2008 (2008-07-03), pages 507-526, XP055136500, ISSN: 0273-2289, DOI: 10.1007/s12010-008-8298-9
- HORI KANJI ET AL: "Lectin-like compounds and lectin receptors in marine microalgae: Hemagglutination and reactivity with purified lectins", JOURNAL OF PHYCOL, PHYCOLOGICAL SOCIETY OF AMERICA, NEW YORK, NY, US, vol. 32, no. 5, 1 October 1996 (1996-10-01), pages 783-790, XP009109894, ISSN: 0022-3646, DOI: 10.1111/J.0022-3646.1996.00783.X
- LOPEZ ALONSO D. ET AL.: "FIRST INSIGHTS INTO IMPROVEMENT OF EICOSAPENTAENOIC ACID CONTENT IN PHAEODACTYLUM TRICORNUTUM (BACILLARIOPHYCEAE) BY INDUCED MUTAGENESIS", JOURNAL OF PHYCOLOGY, vol. 32, no. 2, April 2019 (2019-04), pages 339-345,

### SEQUENCE LISTING

<110> SOURDIVE, DAVID
<120> METHOD FOR TARGETED GENOMIC EVENTS IN ALGAE
<130> 364899WO59
<150> US 61/370,017
   <151> 2010-08-02
<160> 93
<170> PatentIn version 3.5
<210> 1
   <211> 163
   <212> PRT
   <213> Chlamydomonas reinhardtii monomer
<400> 1
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Thr Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Glu Gln Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 2
   <211> 167
   <212> PRT
   <213> Chlamydomonas reinhardtii monomer
<400> 2
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asp Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Asp
                   165
<210> 3
   <211> 22
   <212> DNA
   <213> Artificial Sequence
<220>
   <223> C1221 target
<400> 3
   caaaacgtcg tacgacgttt tg 22
<210> 4
   <211> 24
   <212> DNA
   <213> Artificial Sequence
<220>
   <223> STA6.1 target
<400> 4
   tgggcacgac ttgcattgtg tact 24
<210> 5
   <211> 24
   <212> DNA
   <213> Artificial Sequence
<220>
   <223> STA6.2 target
<400> 5
   atttactgcc tcacccagtt caac 24
<210> 6
   <211> 24
   <212> DNA
   <213> Artificial Sequence
<220>
   <223> STA6.3 target
<400> 6
   tcccgctagg gcacaggagc gaac 24
<210> 7
   <211> 24
   <212> DNA
   <213> Artificial Sequence
<220>
   <223> STA6.4 target
<400> 7
   accgcacacc gtaccgcgtc caca 24
<210> 8
   <211> 24
   <212> DNA
   <213> Artificial Sequence
<220>
   <223> STA6.5 target
<400> 8
   gtccgccaac aagagctggt tcca 24
<210> 9
   <211> 24
   <212> DNA
   <213> Artificial Sequence
<220>
   <223> STA6.6 target
<400> 9
   ttcctcctgc gcaaggcagc gagg 24
<210> 10
   <211> 24
   <212> DNA
   <213> Artificial Sequence
<220>
   <223> STA6.7 target
<400> 10
   tgaggccgcc gtaactgggg gtgg 24
<210> 11
   <211> 24
   <212> DNA
   <213> Artificial Sequence
<220>
   <223> STA6.8 target
<400> 11
   gtgccactgg gcacggtggc ctgc 24
<210> 12
   <211> 24
   <212> DNA
   <213> Artificial Sequence
<220>
   <223> STA6.9 target
<400> 12
   acgcgctggt acacggaggg ctac 24
<210> 13
   <211> 24
   <212> DNA
   <213> Artificial Sequence
<220>
   <223> STA6.10 target
<400> 13
   tggggatatg gttccagggc taat 24
<210> 14
   <211> 24
   <212> DNA
   <213> Artificial Sequence
<220>
   <223> STA6.11 target
<400> 14
   ctgggatggg tcaaggtgga gggg 24
<210> 15
   <211> 24
   <212> DNA
   <213> Artificial Sequence
<220>
   <223> STA6.12 target
<400> 15
   acgcgccgat ctacaccatg tcgc 24
<210> 16
   <211> 24
   <212> DNA
   <213> Artificial Sequence
<220>
   <223> STA6.13 target
<400> 16
   tcgggccggg aagaggcggc gcgg 24
<210> 17
   <211> 24
   <212> DNA
   <213> Artificial Sequence
<220>
   <223> STA6.14 target
<400> 17
   cttggctgcg tttttgggtt ggaa 24
<210> 18
   <211> 24
   <212> DNA
   <213> Artificial Sequence
<220>
   <223> STA6.15 target
<400> 18
   actctataga gtagggggga ttga 24
<210> 19
   <211> 24
   <212> DNA
   <213> Artificial Sequence
<220>
   <223> STA6.16 target
<400> 19
   gtgggatgcc gtaggagggg cggg 24
<210> 20
   <211> 24
   <212> DNA
   <213> Artificial Sequence
<220>
   <223> chr_2 target
<400> 20
   tcgtgatgct gtaaaggatt ttga 24
<210> 21
   <211> 24
   <212> DNA
   <213> Artificial Sequence
<220>
   <223> chr_2 target
<400> 21
   ttttgacgtc gtacggtgtc tccg 24
<210> 22
   <211> 24
   <212> DNA
   <213> Artificial Sequence
<220>
   <223> chr_4 target
<400> 22
   gaaggatacc gtaagtaggt ttgg 24
<210> 23
   <211> 24
   <212> DNA
   <213> Artificial Sequence
<220>
   <223> chr_4 target
<400> 23
   acaagccatt gtacgttgtt ccgt 24
<210> 24
   <211> 24
   <212> DNA
   <213> Artificial Sequence
<220>
   <223> chr_4 target
<400> 24
   attgaatctt ttacaagagc aagg 24
<210> 25
   <211> 24
   <212> DNA
   <213> Artificial Sequence
<220>
   <223> chr_7 target
<400> 25
   ttttgctctt gtacggcgtc ctgg 24
<210> 26
   <211> 24
   <212> DNA
   <213> Artificial Sequence
<220>
   <223> chr_8 target
<400> 26
   tcaaaacttt gtacagaatg ttgt 24
<210> 27
   <211> 24
   <212> DNA
   <213> Artificial Sequence
<220>
   <223> chr_9 target
<400> 27
   accagacccc gtaaaagaga tgga 24
<210> 28
   <211> 24
   <212> DNA
   <213> Artificial Sequence
<220>
   <223> chr_9 target
<400> 28
   acgaaactac gtactccatt ttgg 24
<210> 29
   <211> 24
   <212> DNA
   <213> Artificial Sequence
<220>
   <223> chr_12 target
<400> 29
   gaaaactgtc gtacagggtc tcga 24
<210> 30
   <211> 24
   <212> DNA
   <213> Artificial Sequence
<220>
   <223> chr_16 target
<400> 30
   gcaaactctg ttacatagta caac 24
<210> 31
   <211> 24
   <212> DNA
   <213> Artificial Sequence
<220>
   <223> chr_17 target
<400> 31
   gcaaactgag ttaagggatc cgaa 24
<210> 32
   <211> 24
   <212> DNA
   <213> Artificial Sequence
<220>
   <223> chr_18 target
<400> 32
   ccgagaccct gtaaaaggtg gaag 24
<210> 33
   <211> 24
   <212> DNA
   <213> Artificial Sequence
<220>
   <223> chr_18 target
<400> 33
   cctggccctg gtaaatagtc ttgg 24
<210> 34
   <211> 24
   <212> DNA
   <213> Artificial Sequence
<220>
   <223> chr_18 target
<400> 34
   tttgtctttt gtaagacaga caat 24
<210> 35
   <211> 24
   <212> DNA
   <213> Artificial Sequence
<220>
   <223> chr_19 target
<400> 35
   acaaactggc gtaacgcagt ttat 24
<210> 36
   <211> 24
   <212> DNA
   <213> Artificial Sequence
<220>
   <223> chr_20 target
<400> 36
   ccaaaccgtt ttacagtata ttca 24
<210> 37
   <211> 24
   <212> DNA
   <213> Artificial Sequence
<220>
   <223> chr_21 target
<400> 37
   acgggactac gtacgacgga atgc 24
<210> 38
   <211> 24
   <212> DNA
   <213> Artificial Sequence
<220>
   <223> chr_21 target
<400> 38
   gcaaaatggt ttacgacagt acga 24
<210> 39
   <211> 24
   <212> DNA
   <213> Artificial Sequence
<220>
   <223> chr_28 target
<400> 39
   gttttacgtt gtacgacgtc tagc 24
<210> 40
   <211> 24
   <212> DNA
   <213> Artificial Sequence
<220>
   <223> chr_2 target
<400> 40
   tccttcttcg gtacgtcatt ttct 24
<210> 41
   <211> 24
   <212> DNA
   <213> Artificial Sequence
<220>
   <223> chr_3 target
<400> 41
   acaagacgtc tcacgttgtt ccgt 24
<210> 42
   <211> 24
   <212> DNA
   <213> Artificial Sequence
<220>
   <223> chr_3 target
<400> 42
   tttggccgag gtacagggta caaa 24
<210> 43
   <211> 24
   <212> DNA
   <213> Artificial Sequence
<220>
   <223> chr_3 target
<400> 43
   gctacatgtc gtaaccagta cgga 24
<210> 44
   <211> 24
   <212> DNA
   <213> Artificial Sequence
<220>
   <223> chr_4 target
<400> 44
   acaatatggc ttacaaaaga cagg 24
<210> 45
   <211> 24
   <212> DNA
   <213> Artificial Sequence
<220>
   <223> chr_5 target
<400> 45
   acaagctatt ttacaagggt cggc 24
<210> 46
   <211> 24
   <212> DNA
   <213> Artificial Sequence
<220>
   <223> chr_6 target
<400> 46
   tccctctctt gtgacaaatg taaa 24
<210> 47
   <211> 24
   <212> DNA
   <213> Artificial Sequence
<220>
   <223> chr_6 target
<400> 47
   tcctgctcct gtaagaaaga ttgc 24
<210> 48
   <211> 24
   <212> DNA
   <213> Artificial Sequence
<220>
   <223> chr_6 target
<400> 48
   ataggatcca tcacgacgtt tcac 24
<210> 49
   <211> 24
   <212> DNA
   <213> Artificial Sequence
<220>
   <223> chr_8 target
<400> 49
   actggccgat gtaagacgtc caac 24
<210> 50
   <211> 24
   <212> DNA
   <213> Artificial Sequence
<220>
   <223> chr_8 target
<400> 50
   gtgaaactat gtaaaaaggc aatt 24
<210> 51
   <211> 24
   <212> DNA
   <213> Artificial Sequence
<220>
   <223> chr_13 target
<400> 51
   atgaaacgac gtacgaagta ttgg 24
<210> 52
   <211> 24
   <212> DNA
   <213> Artificial Sequence
<220>
   <223> chr_13 target
<400> 52
   tcaatctcag gtgaaacaga gcgt 24
<210> 53
   <211> 24
   <212> DNA
   <213> Artificial Sequence
<220>
   <223> chr_14 target
<400> 53
   tctggctctg gtacgttatc ttgt 24
<210> 54
   <211> 24
   <212> DNA
   <213> Artificial Sequence
<220>
   <223> chr_15 target
<400> 54
   atgacatccg ttaccgaaga agat 24
<210> 55
   <211> 24
   <212> DNA
   <213> Artificial Sequence
<220>
   <223> chr_15 target
<400> 55
   attagacgag gtgagacgtc ttgt 24
<210> 56
   <211> 24
   <212> DNA
   <213> Artificial Sequence
<220>
   <223> chr_16a target
<400> 56
   acaggacgat gtacgacagc gcag 24
<210> 57
   <211> 24
   <212> DNA
   <213> Artificial Sequence
<220>
   <223> chr_17 target
<400> 57
   tcaaccttct ttacaagatc tgac 24
<210> 58
   <211> 24
   <212> DNA
   <213> Artificial Sequence
<220>
   <223> chr_23 target
<400> 58
   gtgatacgtt gtgagaaaga ttaa 24
<210> 59
   <211> 24
   <212> DNA
   <213> Artificial Sequence
<220>
   <223> scaffold_1 target
<400> 59
   attttatgtt gtgaagaagg ttgg 24
<210> 60
   <211> 24
   <212> DNA
   <213> Artificial Sequence
<220>
   <223> scaffold_1 target
<400> 60
   accttctcca ttaccccatc cccc 24
<210> 61
   <211> 24
   <212> DNA
   <213> Artificial Sequence
<220>
   <223> scaffold_12 target
<400> 61
   gcgacctctg gtgaggggtt ttgc 24
<210> 62
   <211> 24
   <212> DNA
   <213> Artificial Sequence
<220>
   <223> scaffold_19 target
<400> 62
   agggcctgtg gtacgacgtc tggg 24
<210> 63
   <211> 24
   <212> DNA
   <213> Artificial Sequence
<220>
   <223> scaffold_20 target
<400> 63
   acaaaacgtc gtacaggatg caag 24
<210> 64
   <211> 24
   <212> DNA
   <213> Artificial Sequence
<220>
   <223> scaffold_20 target
<400> 64
   cctgcctgtt ttaccccgtt ttgg 24
<210> 65
   <211> 24
   <212> DNA
   <213> Artificial Sequence
<220>
   <223> scaffold_24 target
<400> 65
   actgcctgtt ttacaacgtg cagc 24
<210> 66
   <211> 24
   <212> DNA
   <213> Artificial Sequence
<220>
   <223> scaffold_32 target
<400> 66
   acctgcccct gtacccaggc ttgc 24
<210> 67
   <211> 24
   <212> DNA
   <213> Artificial Sequence
<220>
   <223> scaffold_33 target
<400> 67
   attgaacttt gtaagtagtt ttgc 24
<210> 68
   <211> 24
   <212> DNA
   <213> Artificial Sequence
<220>
   <223> scaffold_40 target
<400> 68
   gcttcatcat gtacaacgga tcac 24
<210> 69
   <211> 349
   <212> PRT
   <213> Artificial Sequence
<220>
   <223> TP7 single chain ORF
<400> 69
   Met Ala Asn Thr Lys Tyr Asn Glu Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Thr Lys Phe Lys His Ala Leu Lys Leu Thr Phe Asn Val Thr Gln
           35                  40                  45
   Lys Thr Gln Arg Arg Trp Phe Leu Asp Lys Leu Val Asp Glu Ile Gly
       50                  55                  60
   Val Gly Tyr Val Tyr Asp Ser Gly Ser Val Ser Tyr Tyr Asn Leu Ser
   65                  70                  75                  80
   Glu Ile Lys Pro Leu His Asn Phe Leu Thr Gln Leu Gln Pro Phe Leu
                   85                  90                  95
   Glu Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Val Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Gly Asp Ser Ser Val Ser Asn Ser Glu His
                   165                 170                 175
   Ile Ala Pro Leu Ser Leu Pro Ser Ser Pro Pro Ser Val Gly Ser Asn
               180                 185                 190
   Lys Lys Phe Leu Leu Tyr Leu Ala Gly Phe Val Asp Ser Asp Gly Ser
           195                 200                 205
   Ile Ile Ala Gln Ile Gln Pro Asn Gln Ser Ser Lys Phe Lys His Arg
       210                 215                 220
   Leu Lys Leu Thr Phe Lys Val Thr Gln Lys Thr Gln Arg Arg Trp Leu
   225                 230                 235                 240
   Leu Asp Lys Leu Val Asp Arg Ile Gly Val Gly Tyr Val Glu Asp Ser
                   245                 250                 255
   Gly Ser Val Ser Asn Tyr Arg Leu Ser Glu Ile Lys Pro Leu His Asn
               260                 265                 270
   Phe Leu Thr Gln Leu Gln Pro Phe Leu Lys Leu Lys Gln Lys Gln Ala
           275                 280                 285
   Asn Leu Val Leu Lys Ile Ile Glu Gln Leu Pro Ser Ala Lys Glu Ser
       290                 295                 300
   Pro Asp Lys Phe Leu Glu Val Cys Thr Trp Val Asp Gln Val Ala Ala
   305                 310                 315                 320
   Leu Asn Asp Ser Lys Thr Arg Lys Thr Thr Ser Glu Thr Val Arg Ala
                   325                 330                 335
   Val Leu Asp Ser Leu Ser Glu Lys Lys Lys Ser Ser Pro
               340                 345
<210> 70
   <211> 3829
   <212> DNA
   <213> Artificial Sequence
<220>
   <223> pCLS7126
<400> 70
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgaatt cgagctcggt acctcgcgaa    420
   tgcatctaga tattgggagc tcgtctagag gatcgctcga gttactaagg agaggacttt    480
   ttcttctcag agaggctatc cagaactgcc ctcacagtct cagaggtggt ttttctggtc    540
   ttggagtcat tcaaggcagc aacctgatcc acccaagtac acacttcaag aaacttgtca    600
   ggggactcct tggcagatgg cagttgctcg atgattttca aaaccagatt tgcttgcttc    660
   tgtttgagct tcaagaaggg ttgcagttgg gtgagaaagt tatgaagagg cttaatttca    720
   gacagacggt agtttgacac agagccagag tcttcgacat agcccacacc aatacgatca    780
   accaatttgt ccaacagcca ccttctttgt gtcttctgag tgactttaaa ggtcaatttg    840
   agacggtgtt tgaacttaga agattgattt ggctgtatct gagcaatgat ggagccatca    900
   gaatccacaa atccagcaag atacagcagg aattttttgt tagaaccaac agatggagga    960
   gaggaaggca gagacagagg agcaatgtgc tcggaattag aaacagagga atcaccggcc   1020
   gccggggagg atttcttctt ctcgctcagg ctgtccagca cagcacgaac ggtttcagaa   1080
   gtggttttac gcgtcttaga atcgttcaga gctgcaacct gatccaccca ggtacaaact   1140
   tccaggaatt tgtccgggga ttcttttgca gacggcagct gttcgataat tttcagaacc   1200
   aggtttgcct gtttctgttt cagttccaga aacggctgca gttgagtcag gaagttgtgc   1260
   agcggcttga tttcgcttaa gttgtagtag gaaacgctac cagaatcgta tacgtaacca   1320
   acgccaattt catccactag tttgtccaga aaccaacggc gctgggtctt ttgagtcacg   1380
   ttaaaggtca attttagagc atgtttaaac ttggtagact ggtttggttt aatctgagcg   1440
   atgatgctac cgtcaccgtc cacaaagccg gccaggtaca gcaggaactc ttcgttatat   1500
   ttggtattgg ccatggtggc aaggccgctg tgtaggcgcg ccattgggtc atcggatccc   1560
   gggcccgtcg actgcagagg cctgcatgca agcttggcgt aatcatggtc atagctgttt   1620
   cctgtgtgaa attgttatcc gctcacaatt ccacacaaca tacgagccgg aagcataaag   1680
   tgtaaagcct ggggtgccta atgagtgagc taactcacat taattgcgtt gcgctcactg   1740
   cccgctttcc agtcgggaaa cctgtcgtgc cagctgcatt aatgaatcgg ccaacgcgcg   1800
   gggagaggcg gtttgcgtat tgggcgctct tccgcttcct cgctcactga ctcgctgcgc   1860
   tcggtcgttc ggctgcggcg agcggtatca gctcactcaa aggcggtaat acggttatcc   1920
   acagaatcag gggataacgc aggaaagaac atgtgagcaa aaggccagca aaaggccagg   1980
   aaccgtaaaa aggccgcgtt gctggcgttt ttccataggc tccgcccccc tgacgagcat   2040
   cacaaaaatc gacgctcaag tcagaggtgg cgaaacccga caggactata aagataccag   2100
   gcgtttcccc ctggaagctc cctcgtgcgc tctcctgttc cgaccctgcc gcttaccgga   2160
   tacctgtccg cctttctccc ttcgggaagc gtggcgcttt ctcatagctc acgctgtagg   2220
   tatctcagtt cggtgtaggt cgttcgctcc aagctgggct gtgtgcacga accccccgtt   2280
   cagcccgacc gctgcgcctt atccggtaac tatcgtcttg agtccaaccc ggtaagacac   2340
   gacttatcgc cactggcagc agccactggt aacaggatta gcagagcgag gtatgtaggc   2400
   ggtgctacag agttcttgaa gtggtggcct aactacggct acactagaag aacagtattt   2460
   ggtatctgcg ctctgctgaa gccagttacc ttcggaaaaa gagttggtag ctcttgatcc   2520
   ggcaaacaaa ccaccgctgg tagcggtggt ttttttgttt gcaagcagca gattacgcgc   2580
   agaaaaaaag gatctcaaga agatcctttg atcttttcta cggggtctga cgctcagtgg   2640
   aacgaaaact cacgttaagg gattttggtc atgagattat caaaaaggat cttcacctag   2700
   atccttttaa attaaaaatg aagttttaaa tcaatctaaa gtatatatga gtaaacttgg   2760
   tctgacagtt accaatgctt aatcagtgag gcacctatct cagcgatctg tctatttcgt   2820
   tcatccatag ttgcctgact ccccgtcgtg tagataacta cgatacggga gggcttacca   2880
   tctggcccca gtgctgcaat gataccgcga gacccacgct caccggctcc agatttatca   2940
   gcaataaacc agccagccgg aagggccgag cgcagaagtg gtcctgcaac tttatccgcc   3000
   tccatccagt ctattaattg ttgccgggaa gctagagtaa gtagttcgcc agttaatagt   3060
   ttgcgcaacg ttgttgccat tgctacaggc atcgtggtgt cacgctcgtc gtttggtatg   3120
   gcttcattca gctccggttc ccaacgatca aggcgagtta catgatcccc catgttgtgc   3180
   aaaaaagcgg ttagctcctt cggtcctccg atcgttgtca gaagtaagtt ggccgcagtg   3240
   ttatcactca tggttatggc agcactgcat aattctctta ctgtcatgcc atccgtaaga   3300
   tgcttttctg tgactggtga gtactcaacc aagtcattct gagaatagtg tatgcggcga   3360
   ccgagttgct cttgcccggc gtcaatacgg gataataccg cgccacatag cagaacttta   3420
   aaagtgctca tcattggaaa acgttcttcg gggcgaaaac tctcaaggat cttaccgctg   3480
   ttgagatcca gttcgatgta acccactcgt gcacccaact gatcttcagc atcttttact   3540
   ttcaccagcg tttctgggtg agcaaaaaca ggaaggcaaa atgccgcaaa aaagggaata   3600
   agggcgacac ggaaatgttg aatactcata ctcttccttt ttcaatatta ttgaagcatt   3660
   tatcagggtt attgtctcat gagcggatac atatttgaat gtatttagaa aaataaacaa   3720
   ataggggttc cgcgcacatt tccccgaaaa gtgccacctg acgtctaaga aaccattatt   3780
   atcatgacat taacctataa aaataggcgt atcacgaggc cctttcgtc              3829
<210> 71
   <211> 4951
   <212> DNA
   <213> Artificial Sequence
<220>
   <223> pThpse-LHCF9p-TP7-LHCF9-3' expression plasmid
<400> 71
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgat gcatccgtta acaccggtaa gcggccgcgc tagggataac agggtaatat 240
   tcaaaacgtc gtacgacgtt ttgacctgca ggcgcttttt ccgagaactc cccataagtc 300
   aacggctcca atcaagaatg tatccgacaa cggcgagcat agcaacacgt ccgtctttgg 360
   agtagaatca tcatgttgtg gatgaataca cagatgaatg acattaaaag catgaacatg 420
   ttagagagta ggaggtagag attgatatgg tagcattgcg atgtttgttt ttggtcagca 480
   tatgatgagt ggataccaat atgatgaaag ttgaatctcg cgtttgagct cagcggtacg 540
   ttattgatcg aaagtagcct gatcaaaatc cttggagagt acaagaggat caaagaatcc 600
   agtgggggcg ataactccaa gctcgttctc aaagaggcaa tggaggtaga aactcatccc 660
   agttgagaag aagtgaaggc agtggcggtg gcgaaagcag aggcaacgag gacagacttc 720
   ctgtgggttg atgcaacgaa tatttccaga aggagaagtt tagagagttg aaccgctacc 780
   tacaatgaca aagtatcgta tcgattttga tgttggttgg ttatgaattc aaactgtaag 840
   ttggattgtg agaagatcag aagttgaacg aacacatctt tccgatcatt cacctccaca 900
   ctgcaacaac acggtacttc ttccgcggca ggtctctgtc gccattctct tgtcctgttg 960
   ttggctgtga gacgaggaaa gcaacgacaa gtttcacaaa agggagttcc tttaacgaga 1020
   tatgtttttt ataaagagtc ccaatagaaa gacaaattga ttcctccgtg caaacgcgca 1080
   aataaacacc acgtccatta tatccatatc tttcagagta tccaacaagt gttgaaggac 1140
   aggtagttga agtaacgtat cttccccctc gactggatcc atcaacaagg cgaacaaatc 1200
   cattcaacct ctcataaatt atctgattta ccaaaccaat accaaattaa ttaagtaggc 1260
   gcgcctacac agcggccttg ccaccatggc caataccaaa tataacgaag agttcctgct 1320
   gtacctggcc ggctttgtgg acggtgacgg tagcatcatc gctcagatta aaccaaacca 1380
   gtctaccaag tttaaacatg ctctaaaatt gacctttaac gtgactcaaa agacccagcg 1440
   ccgttggttt ctggacaaac tagtggatga aattggcgtt ggttacgtat acgattctgg 1500
   tagcgtttcc tactacaact taagcgaaat caagccgctg cacaacttcc tgactcaact 1560
   gcagccgttt ctggaactga aacagaaaca ggcaaacctg gttctgaaaa ttatcgaaca 1620
   gctgccgtct gcaaaagaat ccccggacaa attcctggaa gtttgtacct gggtggatca 1680
   ggttgcagct ctgaacgatt ctaagacgcg taaaaccact tctgaaaccg ttcgtgctgt 1740
   gctggacagc ctgagcgaga agaagaaatc ctccccggcg gccggtgatt cctctgtttc 1800
   taattccgag cacattgctc ctctgtctct gccttcctct cctccatctg ttggttctaa 1860
   caaaaaattc ctgctgtatc ttgctggatt tgtggattct gatggctcca tcattgctca 1920
   gatacagcca aatcaatctt ctaagttcaa acaccgtctc aaattgacct ttaaagtcac 1980
   tcagaagaca caaagaaggt ggctgttgga caaattggtt gatcgtattg gtgtgggcta 2040
   tgtcgaagac tctggctctg tgtcaaacta ccgtctgtct gaaattaagc ctcttcataa 2100
   ctttctcacc caactgcaac ccttcttgaa gctcaaacag aagcaagcaa atctggtttt 2160
   gaaaatcatc gagcaactgc catctgccaa ggagtcccct gacaagtttc ttgaagtgtg 2220
   tacttgggtg gatcaggttg ctgccttgaa tgactccaag accagaaaaa ccacctctga 2280
   gactgtgagg gcagttctgg atagcctctc tgagaagaaa aagtcctctc cttagtaact 2340
   cgagcgatcc tctagctaag atccaatggc aaggaccaag tgctggaact tgttttgctt 2400
   tagcagatct tagcgtgaga ggtatttgtc ctctgtcagg agtagatagt agatgttctt   2460
   tttaaactaa aatgctaact gttccgaatt cctcatcgca gctaatccgt acatcaaaag   2520
   acaaaatgct aggtatgtgt actacatctc ctgttgctag ataagacata tgataggaaa   2580
   cacaccatca atagtcattg tagctttact tatactacgc atttgcactt tcccctgagt   2640
   ggcagaggcg cattgagaaa atcgatctca acatagttta tgtagcatcc cctagatcca   2700
   ttacgttaag tctccttcgt ctttggtgta ggcatgttgg acacaacgag gtaaaacaca   2760
   acacaaacaa tgtgtccagc aaagtagtag ctgctccagt tctcccgttt aaactcactg   2820
   actcgctgcg ctcggtcgtt cggctgcggc gagcggtatc agctcactca aaggcggtaa   2880
   tacggttatc cacagaatca ggggataacg caggaaagac aattgcttat aacacgcgta   2940
   ctagtgctcg cgacgagatc ttacttaagc agtcgacaac ctaggattag cgctccggta   3000
   cctcaaaacg tcgtacgacg ttttgagcta gggataacag ggtaatatgg atccaagata   3060
   tcaagaattc ccatgtgagc aaaaggccag caaaaggcca ggaaccgtaa aaaggccgcg   3120
   ttgctggcgt ttttccatag gctccgcccc cctgacgagc atcacaaaaa tcgacgctca   3180
   agtcagaggt ggcgaaaccc gacaggacta taaagatacc aggcgtttcc ccctggaagc   3240
   tccctcgtgc gctctcctgt tccgaccctg ccgcttaccg gatacctgtc cgcctttctc   3300
   ccttcgggaa gcgtggcgct ttctcatagc tcacgctgta ggtatctcag ttcggtgtag   3360
   gtcgttcgct ccaagctggg ctgtgtgcac gaaccccccg ttcagcccga ccgctgcgcc   3420
   ttatccggta actatcgtct tgagtccaac ccggtaagac acgacttatc gccactggca   3480
   gcagccactg gtaacaggat tagcagagcg aggtatgtag gcggtgctac agagttcttg   3540
   aagtggtggc ctaactacgg ctacactaga aggacagtat ttggtatctg cgctctgctg   3600
   aagccagtta ccttcggaaa aagagttggt agctcttgat ccggcaaaca aaccaccgct   3660
   ggtagcggtg gtttttttgt ttgcaagcag cagattacgc gcagaaaaaa aggatctcaa   3720
   gaagatcctt tgatcttttc tacggggtct gacgctcagt ggaacgaaaa ctcacgttaa   3780
   gggattttgg tcatgagatt atcaaaaagg atcttcacct agatcctttt aaattaaaaa   3840
   tgaagtttta aatcaatcta aagtatatat gagtaaactt ggtctgacag ttaccaatgc   3900
   ttaatcagtg aggcacctat ctcagcgatc tgtctatttc gttcatccat agttgcctga   3960
   ctccccgtcg tgtagataac tacgatacgg gagggcttac catctggccc cagtgctgca   4020
   atgataccgc gagacccacg ctcaccggct ccagatttat cagcaataaa ccagccagcc   4080
   ggaagggccg agcgcagaag tggtcctgca actttatccg cctccatcca gtctattaat   4140
   tgttgccggg aagctagagt aagtagttcg ccagttaata gtttgcgcaa cgttgttgcc   4200
   attgctacag gcatcgtggt gtcacgctcg tcgtttggta tggcttcatt cagctccggt   4260
   tcccaacgat caaggcgagt tacatgatcc cccatgttgt gcaaaaaagc ggttagctcc   4320
   ttcggtcctc cgatcgttgt cagaagtaag ttggccgcag tgttatcact catggttatg   4380
   gcagcactgc ataattctct tactgtcatg ccatccgtaa gatgcttttc tgtgactggt   4440
   gagtactcaa ccaagtcatt ctgagaatag tgtatgcggc gaccgagttg ctcttgcccg   4500
   gcgtcaatac gggataatac cgcgccacat agcagaactt taaaagtgct catcattgga   4560
   aaacgttctt cggggcgaaa actctcaagg atcttaccgc tgttgagatc cagttcgatg   4620
   taacccactc gtgcacccaa ctgatcttca gcatctttta ctttcaccag cgtttctggg   4680
   tgagcaaaaa caggaaggca aaatgccgca aaaaagggaa taagggcgac acggaaatgt   4740
   tgaatactca tactcttcct ttttcaatat tattgaagca tttatcaggg ttattgtctc   4800
   atgagcggat acatatttga atgtatttag aaaaataaac aaataggggt tccgcgcaca   4860
   tttccccgaa aagtgccacc tgacgtctaa gaaaccatta ttatcatgac attaacctat   4920
   aaaaataggc gtatcacgag gccctttcgt c                                  4951
<210> 72
   <211> 4447
   <212> DNA
   <213> Artificial Sequence
<220>
   <223> pThpse-LHCF9p-NAT-LHCF9-3' expression plasmid
<400> 72
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgat gcatccgtta acaccggtaa gcggccgcgc tagggataac agggtaatat     240
   tcaaaacgtc gtacgacgtt ttgacctgca ggcgcttttt ccgagaactc cccataagtc     300
   aacggctcca atcaagaatg tatccgacaa cggcgagcat agcaacacgt ccgtctttgg     360
   agtagaatca tcatgttgtg gatgaataca cagatgaatg acattaaaag catgaacatg     420
   ttagagagta ggaggtagag attgatatgg tagcattgcg atgtttgttt ttggtcagca     480
   tatgatgagt ggataccaat atgatgaaag ttgaatctcg cgtttgagct cagcggtacg 540
   ttattgatcg aaagtagcct gatcaaaatc cttggagagt acaagaggat caaagaatcc 600
   agtgggggcg ataactccaa gctcgttctc aaagaggcaa tggaggtaga aactcatccc 660
   agttgagaag aagtgaaggc agtggcggtg gcgaaagcag aggcaacgag gacagacttc 720
   ctgtgggttg atgcaacgaa tatttccaga aggagaagtt tagagagttg aaccgctacc 780
   tacaatgaca aagtatcgta tcgattttga tgttggttgg ttatgaattc aaactgtaag 840
   ttggattgtg agaagatcag aagttgaacg aacacatctt tccgatcatt cacctccaca 900
   ctgcaacaac acggtacttc ttccgcggca ggtctctgtc gccattctct tgtcctgttg 960
   ttggctgtga gacgaggaaa gcaacgacaa gtttcacaaa agggagttcc tttaacgaga 1020
   tatgtttttt ataaagagtc ccaatagaaa gacaaattga ttcctccgtg caaacgcgca 1080
   aataaacacc acgtccatta tatccatatc tttcagagta tccaacaagt gttgaaggac 1140
   aggtagttga agtaacgtat cttccccctc gactggatcc atcaacaagg cgaacaaatc 1200
   cattcaacct ctcataaatt atctgattta ccaaaccaat accaaattaa ttaaatgacc 1260
   actcttgacg acacggctta ccggtaccgc accagtgtcc cgggggacgc cgaggccatc 1320
   gaggcactgg atgggtcctt caccaccgac accgtcttcc gcgtcaccgc caccggggac 1380
   ggcttcaccc tgcgggaggt gccggtggac ccgcccctga ccaaggtgtt ccccgacgac 1440
   gaatcggacg acgaatcgga cgacggggag gacggcgacc cggactcccg gacgttcgtc 1500
   gcgtacgggg acgacggcga cctggcgggc ttcgtggtca tctcgtactc ggcgtggaac 1560
   cgccggctga ccgtcgagga catcgaggtc gccccggagc accgggggca cggggtcggg 1620
   cgcgcgttga tggggctcgc gacggagttc gccggcgagc ggggcgccgg gcacctctgg 1680
   ctggaggtca ccaacgtcaa cgcaccggcg atccacgcgt accggcggat ggggttcacc 1740
   ctctgcggcc tggacaccgc cctgtacgac ggcaccgcct cggacggcga gcggcaggcg 1800
   ctctacatga gcatgccctg cccctgaggc gcgccttaac atgtttgcta gctaagatcc 1860
   aatggcaagg accaagtgct ggaacttgtt ttgctttagc agatcttagc gtgagaggta 1920
   tttgtcctct gtcaggagta gatagtagat gttcttttta aactaaaatg ctaactgttc 1980
   cgaattcctc atcgcagcta atccgtacat caaaagacaa aatgctaggt atgtgtacta 2040
   catctcctgt tgctagataa gacatatgat aggaaacaca ccatcaatag tcattgtagc 2100
   tttacttata ctacgcattt gcactttccc ctgagtggca gaggcgcatt gagaaaatcg 2160
   atctcaacat agtttatgta gcatccccta gatccattac gttaagtctc cttcgtcttt 2220
   ggtgtaggca tgttggacac aacgaggtaa aacacaacac aaacaatgtg tccagcaaag 2280
   tagtagctgc tccagttctc ccgtttaaac tcactgactc gctgcgctcg gtcgttcggc 2340
   tgcggcgagc ggtatcagct cactcaaagg cggtaatacg gttatccaca gaatcagggg 2400
   ataacgcagg aaagacaatt gcttataaca cgcgtactag tgctcgcgac gagatcttac 2460
   ttaagcagtc gacaacctag gattagcgct ccggtacctc aaaacgtcgt acgacgtttt 2520
   gagctaggga taacagggta atatggatcc aagatatcaa gaattcccat gtgagcaaaa 2580
   ggccagcaaa aggccaggaa ccgtaaaaag gccgcgttgc tggcgttttt ccataggctc 2640
   cgcccccctg acgagcatca caaaaatcga cgctcaagtc agaggtggcg aaacccgaca 2700
   ggactataaa gataccaggc gtttccccct ggaagctccc tcgtgcgctc tcctgttccg 2760
   accctgccgc ttaccggata cctgtccgcc tttctccctt cgggaagcgt ggcgctttct 2820
   catagctcac gctgtaggta tctcagttcg gtgtaggtcg ttcgctccaa gctgggctgt 2880
   gtgcacgaac cccccgttca gcccgaccgc tgcgccttat ccggtaacta tcgtcttgag 2940
   tccaacccgg taagacacga cttatcgcca ctggcagcag ccactggtaa caggattagc 3000
   agagcgaggt atgtaggcgg tgctacagag ttcttgaagt ggtggcctaa ctacggctac 3060
   actagaagga cagtatttgg tatctgcgct ctgctgaagc cagttacctt cggaaaaaga 3120
   gttggtagct cttgatccgg caaacaaacc accgctggta gcggtggttt ttttgtttgc 3180
   aagcagcaga ttacgcgcag aaaaaaagga tctcaagaag atcctttgat cttttctacg 3240
   gggtctgacg ctcagtggaa cgaaaactca cgttaaggga ttttggtcat gagattatca 3300
   aaaaggatct tcacctagat ccttttaaat taaaaatgaa gttttaaatc aatctaaagt 3360
   atatatgagt aaacttggtc tgacagttac caatgcttaa tcagtgaggc acctatctca 3420
   gcgatctgtc tatttcgttc atccatagtt gcctgactcc ccgtcgtgta gataactacg 3480
   atacgggagg gcttaccatc tggccccagt gctgcaatga taccgcgaga cccacgctca 3540
   ccggctccag atttatcagc aataaaccag ccagccggaa gggccgagcg cagaagtggt 3600
   cctgcaactt tatccgcctc catccagtct attaattgtt gccgggaagc tagagtaagt 3660
   agttcgccag ttaatagttt gcgcaacgtt gttgccattg ctacaggcat cgtggtgtca 3720
   cgctcgtcgt ttggtatggc ttcattcagc tccggttccc aacgatcaag gcgagttaca 3780
   tgatccccca tgttgtgcaa aaaagcggtt agctccttcg gtcctccgat cgttgtcaga 3840
   agtaagttgg ccgcagtgtt atcactcatg gttatggcag cactgcataa ttctcttact 3900
   gtcatgccat ccgtaagatg cttttctgtg actggtgagt actcaaccaa gtcattctga 3960
   gaatagtgta tgcggcgacc gagttgctct tgcccggcgt caatacggga taataccgcg 4020
   ccacatagca gaactttaaa agtgctcatc attggaaaac gttcttcggg gcgaaaactc 4080
   tcaaggatct taccgctgtt gagatccagt tcgatgtaac ccactcgtgc acccaactga 4140
   tcttcagcat cttttacttt caccagcgtt tctgggtgag caaaaacagg aaggcaaaat 4200
   gccgcaaaaa agggaataag ggcgacacgg aaatgttgaa tactcatact cttccttttt 4260
   caatattatt gaagcattta tcagggttat tgtctcatga gcggatacat atttgaatgt   4320
   atttagaaaa ataaacaaat aggggttccg cgcacatttc cccgaaaagt gccacctgac   4380
   gtctaagaaa ccattattat catgacatta acctataaaa ataggcgtat cacgaggccc   4440
   tttcgtc                                                             4447
<210> 73
   <211> 6211
   <212> DNA
   <213> Artificial Sequence
<220>
   <223> TP7-KI matrix
<400> 73
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgat gcatccgtta acaccggtaa gcggccgcca agcttcattt gttggccgac    240
   aagtgcttgc ctcccatttt ggaagtcatg aatggattgg tgggagggaa tgacgggaat    300
   gagaacgagg actgtgcatt acgagctagt ttggaggcat tggaaaagac cgcagaggaa    360
   ctgtccaagg gggtgagcct tgctgaagca gtgattgact ttgattctgc tccccgcgac    420
   tttatcgtga atccaaacct tgacagcaac cttggcaacg tcaaggctga attggatggt    480
   attcagcagg agctggagga gatccacgca gaaatgaatg ctgcatggta tgaagtgagc    540
   aaggagggag gcatgcaaca agttcggctg gaggatgttg actcaaatag caacacttct    600
   tgtgtgtggc agtttcgtct acccaaaacc aatgacgcga agatgttgac ggattctttt    660
   gattccgtta agatccatcg tattttgaag aacggaggta cgtctcgctt tgagtcagca    720
   ttgttttggg agagcgtcca gatattgatg tgtgtctaac tactaactac tctttttcgc    780
   tgcttagtgt atttctccac gaaggagttg gaacagctcg gcacaaagaa gaaggatttg    840
   atgatggagt acgaggagaa gcaacgtgat attgtatgca aggccatggt tgtggctgca    900
   agttacgtgc cagtgttgga acgagcatcg atgactttat ctgagttgga tgtattggcg    960
   agctttgctt atgtggctgc atacagtagc aacggatact gtcgtcctga gatgacggac   1020
   ggagaagagg acggattggg aattgaggtt agttattcga gcaccgaacg ttgcgattct   1080
   tcgctttggt tctctaaaca caacatatct tttctcgtca atacatttca gctcacgggg   1140
   gctcgtcact gcaggcgctt tttccgagaa ctccccataa gtcaacggct ccaatcaaga   1200
   atgtatccga caacggcgag catagcaaca cgtccgtctt tggagtagaa tcatcatgtt   1260
   gtggatgaat acacagatga atgacattaa aagcatgaac atgttagaga gtaggaggta   1320
   gagattgata tggtagcatt gcgatgtttg tttttggtca gcatatgatg agtggatacc   1380
   aatatgatga aagttgaatc tcgcgtttga gctcagcggt acgttattga tcgaaagtag   1440
   cctgatcaaa atccttggag agtacaagag gatcaaagaa tccagtgggg gcgataactc   1500
   caagctcgtt ctcaaagagg caatggaggt agaaactcat cccagttgag aagaagtgaa   1560
   ggcagtggcg gtggcgaaag cagaggcaac gaggacagac ttcctgtggg ttgatgcaac   1620
   gaatatttcc agaaggagaa gtttagagag ttgaaccgct acctacaatg acaaagtatc   1680
   gtatcgattt tgatgttggt tggttatgaa ttcaaactgt aagttggatt gtgagaagat   1740
   cagaagttga acgaacacat ctttccgatc attcacctcc acactgcaac aacacggtac   1800
   ttcttccgcg gcaggtctct gtcgccattc tcttgtcctg ttgttggctg tgagacgagg   1860
   aaagcaacga caagtttcac aaaagggagt tcctttaacg agatatgttt tttataaaga   1920
   gtcccaatag aaagacaaat tgattcctcc gtgcaaacgc gcaaataaac accacgtcca   1980
   ttatatccat atctttcaga gtatccaaca agtgttgaag gacaggtagt tgaagtaacg   2040
   tatcttcccc ctcgactgga tccatcaaca aggcgaacaa atccattcaa cctctcataa   2100
   attatctgat ttaccaaacc aataccaaat taattaaatg accactcttg acgacacggc   2160
   ttaccggtac cgcaccagtg tcccggggga cgccgaggcc atcgaggcac tggatgggtc   2220
   cttcaccacc gacaccgtct tccgcgtcac cgccaccggg gacggcttca ccctgcggga   2280
   ggtgccggtg gacccgcccc tgaccaaggt gttccccgac gacgaatcgg acgacgaatc   2340
   ggacgacggg gaggacggcg acccggactc ccggacgttc gtcgcgtacg gggacgacgg   2400
   cgacctggcg ggcttcgtgg tcatctcgta ctcggcgtgg aaccgccggc tgaccgtcga   2460
   ggacatcgag gtcgccccgg agcaccgggg gcacggggtc gggcgcgcgt tgatggggct   2520
   cgcgacggag ttcgccggcg agcggggcgc cgggcacctc tggctggagg tcaccaacgt   2580
   caacgcaccg gcgatccacg cgtaccggcg gatggggttc accctctgcg gcctggacac   2640
   cgccctgtac gacggcaccg cctcggacgg cgagcggcag gcgctctaca tgagcatgcc   2700
   ctgcccctga ggcgcgcctt aacatgtttg ctagctaaga tccaatggca aggaccaagt   2760
   gctggaactt gttttgcttt agcagatctt agcgtgagag gtatttgtcc tctgtcagga   2820
   gtagatagta gatgttcttt ttaaactaaa atgctaactg ttccgaattc ctcatcgcag   2880
   ctaatccgta catcaaaaga caaaatgcta ggtatgtgta ctacatctcc tgttgctaga   2940
   taagacatat gataggaaac acaccatcaa tagtcattgt agctttactt atactacgca   3000
   tttgcacttt cccctgagtg gcagaggcgc attgagaaaa tcgatctcaa catagtttat   3060
   gtagcatccc ctagatccat tacgttaagt ctccttcgtc tttggtgtag gcatgttgga   3120
   cacaacgagg taaaacacaa cacaaacaat gtgtccagca aagtagtagc tgctccagtt   3180
   ctcccgttta aactcactga ctcgctgcgc tcggtcgttc ggctgcggcg agcggtatca   3240
   gctcactcaa aggcggtaat acggttatcc acagaatcag gggataacgc aggaaagaca   3300
   attctcgctt ggagctatca ttaccttggc gcagattgga agttttgtac cctgtacctc   3360
   ggccaaaatc aacattgttg atcatatctt ggccagagtt ggagctggtg acgcacagga   3420
   tcgtggtata tctaccttca tggctgaaat gctggaggct tcttccattc ttcgcacctc   3480
   gaccaaacgc agtctcatca tcattgacga gctcggaaga ggaacgagca catttgatgg   3540
   atttggtttg gcaaaagcga tatcagaaca tgtcgttcag aaaattggtt gcatgactgt   3600
   gtttgcaact cacttccatg aactgacggc gttggaagag caagaggcct cggtaaccaa   3660
   ttgccacgtg tctgcccaca gcgacaaaca gaacggactt acgtttctct acgaagtacg   3720
   accagggcct tgcttggaaa gttttggtat tcaagtggcc gaaatggcaa acatgccgtc   3780
   aaatatcatc accgatgcca aacgcaaagc aaaacagttg gagaactttg actatcgcaa   3840
   gaaagctaaa gttacggaga aagactgcat cgctgacaac gaggatgatc atgagacgaa   3900
   agcagctgca atggaatttc ttcacaagtt taggaagctt ccagtgaatg aaatgtcgga   3960
   agaagagttg aaggagatag cgcttccttt gctaaggcag tacggatttg aagcgttggg   4020
   gtgaagtgat cttgttcagt ggatctattc ttcatactat ctcttctttt gtagtgtaat   4080
   ccatgtaaga cttgctttta tgatactgac actatctttc agaactttcc gtttgttttg   4140
   cactgtctct ttacgttagg ttgccaagca aaaaaccgtt acctgtccga gccagccccc   4200
   gccaattcac ctgttctcat aaacttaagc agtcgacaac ctaggattag cgctccggta   4260
   cctcaaaacg tcgtacgacg ttttgagcta gggataacag ggtaatatgg atccaagata   4320
   tcaagaattc ccatgtgagc aaaaggccag caaaaggcca ggaaccgtaa aaaggccgcg   4380
   ttgctggcgt ttttccatag gctccgcccc cctgacgagc atcacaaaaa tcgacgctca   4440
   agtcagaggt ggcgaaaccc gacaggacta taaagatacc aggcgtttcc ccctggaagc   4500
   tccctcgtgc gctctcctgt tccgaccctg ccgcttaccg gatacctgtc cgcctttctc   4560
   ccttcgggaa gcgtggcgct ttctcatagc tcacgctgta ggtatctcag ttcggtgtag   4620
   gtcgttcgct ccaagctggg ctgtgtgcac gaaccccccg ttcagcccga ccgctgcgcc   4680
   ttatccggta actatcgtct tgagtccaac ccggtaagac acgacttatc gccactggca   4740
   gcagccactg gtaacaggat tagcagagcg aggtatgtag gcggtgctac agagttcttg   4800
   aagtggtggc ctaactacgg ctacactaga aggacagtat ttggtatctg cgctctgctg   4860
   aagccagtta ccttcggaaa aagagttggt agctcttgat ccggcaaaca aaccaccgct   4920
   ggtagcggtg gtttttttgt ttgcaagcag cagattacgc gcagaaaaaa aggatctcaa   4980
   gaagatcctt tgatcttttc tacggggtct gacgctcagt ggaacgaaaa ctcacgttaa   5040
   gggattttgg tcatgagatt atcaaaaagg atcttcacct agatcctttt aaattaaaaa   5100
   tgaagtttta aatcaatcta aagtatatat gagtaaactt ggtctgacag ttaccaatgc   5160
   ttaatcagtg aggcacctat ctcagcgatc tgtctatttc gttcatccat agttgcctga   5220
   ctccccgtcg tgtagataac tacgatacgg gagggcttac catctggccc cagtgctgca   5280
   atgataccgc gagacccacg ctcaccggct ccagatttat cagcaataaa ccagccagcc   5340
   ggaagggccg agcgcagaag tggtcctgca actttatccg cctccatcca gtctattaat   5400
   tgttgccggg aagctagagt aagtagttcg ccagttaata gtttgcgcaa cgttgttgcc   5460
   attgctacag gcatcgtggt gtcacgctcg tcgtttggta tggcttcatt cagctccggt   5520
   tcccaacgat caaggcgagt tacatgatcc cccatgttgt gcaaaaaagc ggttagctcc   5580
   ttcggtcctc cgatcgttgt cagaagtaag ttggccgcag tgttatcact catggttatg   5640
   gcagcactgc ataattctct tactgtcatg ccatccgtaa gatgcttttc tgtgactggt   5700
   gagtactcaa ccaagtcatt ctgagaatag tgtatgcggc gaccgagttg ctcttgcccg   5760
   gcgtcaatac gggataatac cgcgccacat agcagaactt taaaagtgct catcattgga   5820
   aaacgttctt cggggcgaaa actctcaagg atcttaccgc tgttgagatc cagttcgatg   5880
   taacccactc gtgcacccaa ctgatcttca gcatctttta ctttcaccag cgtttctggg   5940
   tgagcaaaaa caggaaggca aaatgccgca aaaaagggaa taagggcgac acggaaatgt   6000
   tgaatactca tactcttcct ttttcaatat tattgaagca tttatcaggg ttattgtctc   6060
   atgagcggat acatatttga atgtatttag aaaaataaac aaataggggt tccgcgcaca   6120
   tttccccgaa aagtgccacc tgacgtctaa gaaaccatta ttatcatgac attaacctat   6180
   aaaaataggc gtatcacgag gccctttcgt c                                  6211
<210> 74
   <211> 64
   <212> DNA
   <213> Artificial Sequence
<220>
   <223> MID-129-TP7-4Fw primer
<400> 74
   ccatctcatc cctgcgtgtc tccgactcag cagacgtctg gacgcagcat ttagccatga      60
   aggt                                                                    64
<210> 75
   <211> 64
   <212> DNA
   <213> Artificial Sequence
<220>
   <223> MID-130-TP7-6Fw primer
<400> 75
   ccatctcatc cctgcgtgtc tccgactcag cagtactgcg gacgcagcat ttagccatga      60
   aggt                                                                    64
<210> 76
   <211> 64
   <212> DNA
   <213> Artificial Sequence
<220>
   <223> MID-131-TP7-7Fw primer
<400> 76
   ccatctcatc cctgcgtgtc tccgactcag cgacagcgag gacgcagcat ttagccatga      60
   aggt                                                                    64
<210> 77
   <211> 64
   <212> DNA
   <213> Artificial Sequence
<220>
   <223> MID-132-TP7-9Fw primer
<400> 77
   ccatctcatc cctgcgtgtc tccgactcag cgatctgtcg gacgcagcat ttagccatga      60
   aggt                                                                    64
<210> 78
   <211> 64
   <212> DNA
   <213> Artificial Sequence
<220>
   <223> MID-133-TP7-10Fw primer
<400> 78
   ccatctcatc cctgcgtgtc tccgactcag cgcgtgctag gacgcagcat ttagccatga 60
   aggt 64
<210> 79
   <211> 64
   <212> DNA
   <213> Artificial Sequence
<220>
   <223> MID-134-TP7-llFw primer
<400> 79
   ccatctcatc cctgcgtgtc tccgactcag cgctcgagtg gacgcagcat ttagccatga 60
   aggt 64
<210> 80
   <211> 64
   <212> DNA
   <213> Artificial Sequence
<220>
   <223> MID-135-TP7-16Fw primer
<400> 80
   ccatctcatc cctgcgtgtc tccgactcag cgtgatgacg gacgcagcat ttagccatga 60
   aggt 64
<210> 81
   <211> 64
   <212> DNA
   <213> Artificial Sequence
<220>
   <223> MID-136-TP7-17Fw primer
<400> 81
   ccatctcatc cctgcgtgtc tccgactcag ctatgtacag gacgcagcat ttagccatga 60
   aggt 64
<210> 82
   <211> 64
   <212> DNA
   <213> Artificial Sequence
<220>
   <223> MID-137-TP7-19Fw primer
<400> 82
   ccatctcatc cctgcgtgtc tccgactcag ctcgatatag gacgcagcat ttagccatga      60
   aggt                                                                    64
<210> 83
   <211> 64
   <212> DNA
   <213> Artificial Sequence
<220>
   <223> MID-138-TP7-26Fw primer
<400> 83
   ccatctcatc cctgcgtgtc tccgactcag ctcgcacgcg gacgcagcat ttagccatga      60
   aggt                                                                    64
<210> 84
   <211> 64
   <212> DNA
   <213> Artificial Sequence
<220>
   <223> MID-139-TP7-30Fw primer
<400> 84
   ccatctcatc cctgcgtgtc tccgactcag ctgcgtcacg gacgcagcat ttagccatga      60
   aggt                                                                    64
<210> 85
   <211> 64
   <212> DNA
   <213> Artificial Sequence
<220>
   <223> MID-140-TP7-3lFw primer
<400> 85
   ccatctcatc cctgcgtgtc tccgactcag ctgtgcgtcg gacgcagcat ttagccatga      60
   aggt                                                                    64
<210> 86
   <211> 64
   <212> DNA
   <213> Artificial Sequence
<220>
   <223> MID-141-TP7-32Fw primer
<400> 86
   ccatctcatc cctgcgtgtc tccgactcag tagcatactg gacgcagcat ttagccatga 60
   aggt 64
<210> 87
   <211> 64
   <212> DNA
   <213> Artificial Sequence
<220>
   <223> MID-142-TP7-43Fw primer
<400> 87
   ccatctcatc cctgcgtgtc tccgactcag tatacatgtg gacgcagcat ttagccatga 60
   aggt 64
<210> 88
   <211> 64
   <212> DNA
   <213> Artificial Sequence
<220>
   <223> MID-143-TP7-wtFw primer
<400> 88
   ccatctcatc cctgcgtgtc tccgactcag tatcactcag gacgcagcat ttagccatga 60
   aggt 64
<210> 89
   <211> 50
   <212> DNA
   <213> Artificial Sequence
<220>
   <223> DeepTP7Rv primer
<400> 89
   cctatcccct gtgtgccttg gcagtctcag agctcacgcg ggctcgtcat 50
<210> 90
   <211> 32
   <212> DNA
   <213> Artificial Sequence
<220>
   <223> NotI-TP7LH-Fw primer
<400> 90
   atatgcggcc gccaagcttc atttgttggc cg 32
<210> 91
   <211> 30
   <212> DNA
   <213> Artificial Sequence
<220>
   <223> PstI-TP7LH-Rv primer
<400> 91
   ttaactgcag tgacgagccc ccgtgagctg 30
<210> 92
   <211> 30
   <212> DNA
   <213> Artificial Sequence
<220>
   <223> EcoRI-TP7RH-Fw primer
<400> 92
   atatgaattc tcgcttggag ctatcattac 30
<210> 93
   <211> 32
   <212> DNA
   <213> Artificial Sequence
<220>
   <223> AflII-TP7RH-Rv primer
<400> 93
   ttaacttaag atgagaacag gtgaattggc gg 32

## Description

### BACKGROUND OF THE INVENTION

### Field of the Invention

The description relates to endonucleases cleaving DNA target sequences from diatom genomes, to appropriate vectors encoding such endonucleases, to diatoms modified by such vectors and to the use of these endonucleases and products derived therefrom for targeted genomic engineering in diatoms.

### Discussion of the Background

Although algae have been used as a food source by humans for centuries, the significance of their biotechnological interest, especially of microalgae, appeared only in recent decades. Applications of algal products range from simple biomass production for food, feed and fuels to valuable products such as cosmetics, pharmaceuticals, pigments, sugar polymers and food supplements. For most of these applications, the market is still developing and considering the enormous biodiversity of microalgae and development of genetic engineering, this group of organisms represents one of the most promising sources of new products and applications.

Algae can be found in nearly all aquatic and terrestrial ecosystems, most of them being poorly investigated at the biochemical level, while showing a huge biodiversity and various morphologies ranging from picoplankton species to large kelp (Norton, T. A., Melkonian, M., & Andersen, R. A. (1996) Phycologia 35, 308-326).

Several algal species such as *Dunaliella bardawil, Haematococcus pluvialis* and *Chlorella vulgaris* have already been exploited extensively in the past for biotechnological purposes, especially as feed, as a source of pigments like β-carotene or astaxanthin or as food supplements (Steinbrenner, J. & Sandmann, G. (2006) Appl. Environ. Microbiol. 72, 7477-7484; Mogedas, B., Casal, C., Forjan, E., & Vilchez, C. (2009) J Biosci Bioeng 108, 47-51.).

Most of these organisms are green algae that belonging to a group more related to land plants than other algal groups (Palmer, J. D., Soltis, D. E., & Chase, M. W. (2004) Am. J. Bot. 91, 1437-1445).

Chromophytic algae on the other hand only recently moved into the forefront and their biochemistry and genetics have been studied just in the recent years. They comprise important groups like the brown algae, diatoms, xanthophytes, eustigmatophytes and others, but also the colourless oomycetes (Tyler, B. M., Tripathy, S., Zhang, X., Dehal, P., Jiang, R. H. Y., Aerts, A., Arredondo, F. D., Baxter, L., Bensasson, D., Beynon, J. L. et al. (2006) Science 313, 1261-1266.). Research on chromophytic algae received a strong boost after publication of several genomes including those of the diatoms *Thalassiosira pseudonana* (Armbrust, E. V., Berges, J. A., Bowler, C., Green, B. R., Martinez, D., Putnam, N. H., Zhou, S., Allen, A. E., Apt, K. E., Bechner, M. et al. (2004) Science 306, 79-86.) and *Phaeodactylum tricornutum* (Bowler, C., Allen, A. E., Badger, J. H., Grimwood, J., Jabbari, K., Kuo, A., Maheswari, U., Martens, C., Maumus, F., Otillar, R. P. et al. (2008) Nature. 456, 239-244.).

Genomes of other algae such as *Fragilariopsis cylindrus,* the haptophyte *Emiliania huxleyi* (*http:*//*genome.jgi-psf.org*/*Emihul*/*Emihul.home.html*) and the brown alga *Ectocarpus siliculosus* (in preparation, *http:*//*www.cns.fr*/*spip*/*-Ectocarpus-siliculosus-.html*) are presently studied.

With a large set of genes that originate from bacteria by lateral gene transfer (Bowler, C., Allen, A. E., Badger, J. H., Grimwood, J., Jabbari, K., Kuo, A., Maheswari, U., Martens, C., Maumus, F., Otillar, R. P. et al. (2008) Nature. 456, 239-244.), chromophytic algae demonstrate an enormous genetic complexity and metabolic potential.

*Nannochloropsis,* for example, is a genus of algae comprising approximately six species inside the eustigmatophytes group. *Nannochloropsis* is able to build up a high concentration of a range of pigments such as astaxanthin, zeaxanthin and canthaxanthin. These algae have a very simple ultrastructure that is reduced compared to neighbouring taxa. It is considered as a promising alga for industrial applications because of its ability to accumulate high levels of polyunsaturated fatty acids. It is also mainly used as an energy-rich food source for fish larvae and rotifers.

Diatoms, as another example, are one of the most ecologically successful unicellular phytoplankton on the planet, being responsible for approximately 20% of global carbon fixation, representing a major participant in the marine food web. There are two major potential commercial or technological applications of diatoms. First, Diatoms are able to accumulate abundant amounts of lipid suitable for conversion to liquid fuels and because of their high potential to produce large quantities of lipids and good growth efficiencies, they are considered as one of the best classes of algae for renewable biofuel production. Second, Diatoms have a cell wall consisting of silica (silica exoskeletons called frustules) with intricated and ornate structures on the nano- to micro-scale. These structures exceed the diversity and the complexity capable by man-made synthetic approaches, and Diatoms are being developed as a source of materials mainly for nanotechnological applications (Lusic et al Advanced Functional Materials 2006).

Although some genetic tools to explore microalgal technology are available such as several sequences of genomes and the ability to be genetically transformed, few genome engineering tools exist which considerably limits the use of these organisms for various biotechnological applications. With the current Diatom transformation technology, transformed DNA randomly integrates into the genome, which results in different expression levels for different transformants using the same DNA construct; identifying the highest level of expression can require time-consuming and tedious screening methods.

Any commercial or technological application involving algae will be greatly facilitated by having the ability to perform targeted genomic manipulations ranging from targeted insertions in chosen loci (knock-in), considered or not as "safe harbors" for gene addition (i.e. a loci allowing safe expression of a transgene), to targeted gene knock-out, allele swap, substitutions, marker excisions and deletions, within algae genomes. Moreover, it would be extremely advantageous if these tools allowed targeted genomic manipulations with a high efficacy.

Meganucleases, also referred to as homing endonucleases, were the first endonucleases used to induce double-strand breaks and recombination in living cells (Rouet et al. PNAS 1994 91:6064-6068; Rouet et al. Mol Cell Biol. 1994 14 :8096-8106; Choulika et al. Mol Cell Biol. 1995 15 :1968-1973; Puchta et al. PNAS 1996 93 :5055-5060). However, their use has long been limited by their narrow specificity. Although several hundred natural meganucleases had been identified over the past years, this diversity was still largely insufficient to address genome complexity, and the probability of finding a meganuclease cleavage site within a gene of interest is still extremely low. These findings highlighted the need for artificial endonucleases with tailored specificities, cleaving chosen sequences with the same selectivity as wild-type endonucleases.

Meganucleases have emerged as the scaffolds of choice for creating genome engineering tools cutting a desired target sequence (Paques et al. Curr Gen Ther. 2007 7:49-66). Combinatorial assembly processes allowing to engineer meganucleases with modified specificities has been described by Arnould et al. J Mol Biol. 2006 355:443-458; Arnould et al. J Mol Biol. 2007 371:49-65; Smith et al. NAR 2006 34:e149; Grizot et al. NAR 2009 37:5405). Briefly, these processes rely on the identification of locally engineered variants with a substrate specificity that differs from the substrate specificity of the wild-type meganuclease by only a few nucleotides. Up to four sets of mutations identified in such proteins can then be assembled in new proteins in order to generate new meganucleases with entirely redesigned binding interfaces.

These processes require two steps, wherein different sets of mutations are first assembled into homodimeric variants cleaving palindromic targets. Two homodimers can then be co-expressed in order to generate heterodimeric meganucleases cleaving the chosen non palindromic target. The first step of this process remains the most challenging one, and one cannot know in advance whether a meganuclease cleaving a given locus could be obtained with absolute certainty. Indeed, not all sequences are equally likely to be cleaved by engineered meganucleases, and in certain cases, meganuclease engineering can prove difficult (Galetto et al. Expert Opin Biol Ther. 2009 9:1289-303).

Dürrenberger et al (Nucleic Acid Research, 1996, vol 24, N°17, 3323-3331) discloses the engineering of *Chlamydomonas reinhardtii* chloroplasts, using the wild type endogenous I-CreI endonuclease of this alga. There is however no disclosure of the modification of the nuclear genome of said alga, let alone of a diatom.

De Riso et al (Nucleic Acid Research, 2009, vol 37, N°14, e96) reports the absence of genetic tools to generate loss-of-function mutants in diatoms and discloses the use of the gene silencing technology, by introduction of vectors encoding specific siRNA, for generating knockdown mutants in diatoms.

The inventors have now found new endonucleases cleaving targets within algal diatom genomes that could be used as tools allowing efficient targeted DNA modifications of these genomes, thereby considerably facilitating the handling of these organisms for various biotechnological applications.

### SUMMARY OF THE INVENTION

Therefore, the present invention concerns endonuclease variants cleaving targets within diatom nuclear genomes that could be used as tools allowing efficient targeted genomic engineering of these genomes, thereby considerably facilitating the use of these organisms for various biotechnological applications.

Thus, methods are provided to obtain cultivated diatoms with engineered genomes at specific targeted sites by using endonuclease variants, thereby considerably increasing usability of these organisms for various biotechnological applications. These provided methods range from targeted insertions in chosen loci (knock-in), considered or not as "safe harbors" for gene addition (i.e. a loci allowing safe expression of a transgene), to targeted gene knock-out, allele swap, substitutions, marker excisions, deletions, inside diatom genomes as non-limiting examples of genome engineering.

The above topics highlight certain aspects of the invention. Additional objects, aspects and embodiments of the invention are found in the following detailed description of the invention.

### BRIEF DESCRIPTION OF THE FIGURES

In addition to the preceding features, the invention further comprises other features which will emerge from the description which follows, which refers to examples illustrating endonuclease variants and their uses according to the invention, as well as to the appended drawings. A more complete appreciation of the invention and many of the expected advantages thereof will be readily obtained as the same becomes better understood by reference to the following Figures in conjunction with the detailed description below.
- Figure 1: Illustration of different meganuclease-induced types of recombination events leading to stable and precise genomic modifications. **A.** Targeted integration (insertion of a transgene) through gene conversion; this approach can also be used for gene knock out by homologous recombination. **B.** Knock-out of a gene through Non-Homologous-End-Joining (NHEJ) initiated by a unique double-stranded break. **C.** Knock-out of a gene through NHEJ initiated by two double-stranded breaks (gene excision).

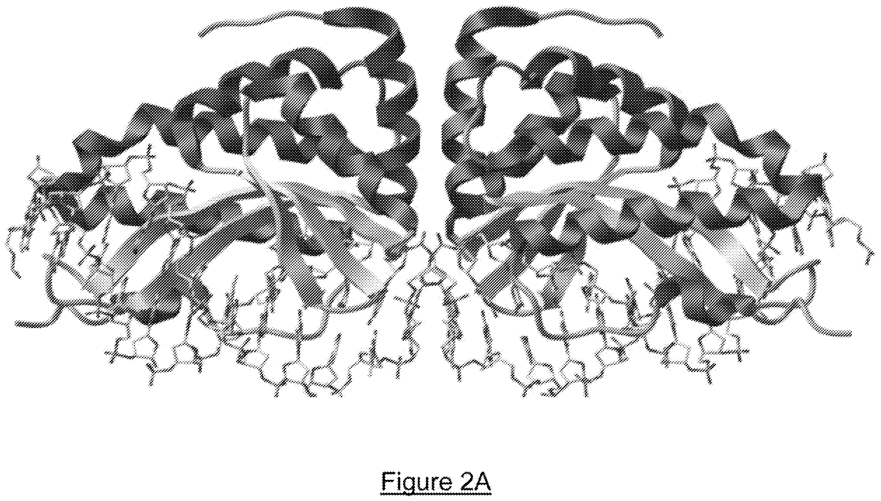

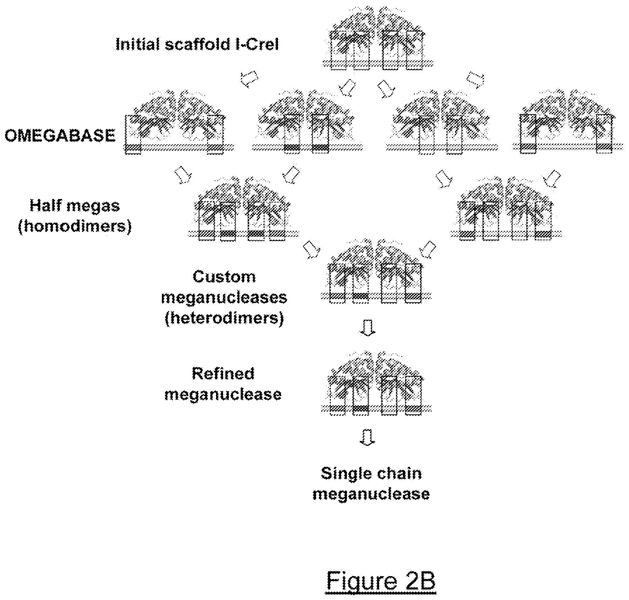

- Figure 2: Modular structure of homing endonucleases and the combinatorial approach for custom meganucleases design **A.** Tridimensional structure of the I-*Cre*I homing endonuclease bound to its DNA target. The catalytic core is surrounded by two αββαββα folds, forming a saddle-shaped interaction interface above the DNA major groove. **B.** A combinatorial process for meganuclease engineering : Four separable DNA binding subdomains (boxed) could be identified in the I-*Cre*I scaffold, an homodimeric meganuclease, that binds and cleaves a palindromic target. Each subdomain can be engineered specifically (boxed), resulting in novel meganucleases cleaving locally altered palindromic targets. Two different subdomains can be combined within a "half meganuclease", a homodimeric meganuclease binding a palindromic target. Two such "half meganucleases" can be co-expressed to form a heterodimeric custom meganuclease that will cleave a novel non palindromic target. Additional steps of engineering (by random or targeted mutagenesis and screening) are often required at this stage to optimize the activity of meganucleases, resulting in a refined meganuclease. In the final version, the two refined monomers can be connected by a linker to make a single-chain meganuclease, as described in Grizot et al. (2009).
- Figure 3: Schematic of the STA6 locus from *Chlamydomonas reinhardtii.* The coding sequences and mRNA sequences are shown, with intervening introns in white. The target positions for 16 meganucleases able to specifically recognize and cleave this locus are also depicted.
- Figure 4: Sequences and locations of targeted sites in the STA6 gene from *Chlamydomonas reinhardtii* (Gene bank accession number: NW001843572).
- Figure 5: Sequences and locations of meganucleases targeted sites in *Phaeodactylum tricornutum* genome (genomic sequences for analysis were found at: http://genome.jgi-psf.org/Phatr2/Phatr2.home.html).
- Figure 6: Sequences and locations of meganucleases targeted sites in *Thalassiosira pseudonana* genome (genomic sequences for analysis were found at:http://genome.jgi-psf.org/Thaps3/Thaps3 .home.html).
- Figure 7: Sequences and locations of meganucleases targeted sites in *Chlorella (NC64A)* genome (genomic sequences for analysis were found at:http://genome.jgi-psf.org/ChlNC64A_1/ChlNC64A_1.home.html).
-Figure 8: Theoretic map of the pThpse-LHCF9p-TP7-LHCF9-3' expression plasmid (SEQ ID NO: 71). LHCF9p = diatom specific promoter region. SC-TP7 = single chain TP7 meganuclease ORF. LHCF9-3' poly(A) signal. I-CreI and I-SceI = target sequences of I-CreI and I-SceI respectively. The rest of the plasmid is a pUC19.
Figure 9: Theoretic map of the pThpse-LHCF9p-NAT-LHCF9-3' expression plasmid (SEQ ID NO: 72). LHCF9p = diatom specific promoter region. natl = natl gene ORF (nourseopthricin acetyl transferase). LHCF9-3' poly(A) signal. I-CreI and I-SceI = target sequences of I-CreI and I-SceI respectively. The rest of the plasmid is a pUC19.
Figure 10: A) TP7-nat selection plate showing positive colonies; B) Agarose gel electrophoresis of 44 PCR colony screenings from TP7 electroporation. White arrowheads indicate colonies used for deep-sequencing assay.
Figure 11: Agarose gel electrophoresis of 13 cDNA amplifications (RT-PCR) showing the presence of full length mRNAs of the MN TP7 in some of the strains. Light and contrast if the picture were modified to detect the paler bands.
Figure 12: Western blot of 13 strains (the strain names are reported below the tracks). As positive control 250 ng of I-CreI purified monomeric protein was used. The band recorded at 40 Kd represents the dimerization of I-CreI. As negative control, *T. pseudonana* wild type protein extract was used.
Figure 13: Theoretic map of the TP7-KI matrix (SEQ ID NO: 73). LHCF9p = diatom specific promoter region. SC-TP7 = single chain TP7 meganuclease ORF (SEQ ID NO: 69). LHCF9-3' poly(A) signal. I-CreI and I-SceI = target sequences of I-CreI and I-SceI respectively. The rest of the plasmid is a pUC19.

### DETAILED DESCRIPTION OF THE INVENTION

Unless specifically defined herein below, all technical and scientific terms used herein have the same meaning as commonly understood by a skilled artisan in the fields of gene therapy, biochemistry, genetics and molecular biology. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

All methods and materials similar or equivalent to those described herein can be used in the practice or testing of the present disclosure, with suitable methods and materials being described herein. In case of conflict, the present specification, including definitions, will overule. Further, the materials, methods, and examples are illustrative only and are not intended to be limiting, unless otherwise specified.

The present invention uses endonuclease variants cleaving targets within nuclear diatom genomes that could be used as tools allowing efficient targeted genomic engineering of these genomes, thereby considerably facilitating the handling of these organisms for various biotechnological applications.

As used herein, the term "endonuclease" refers to any wild-type or variant enzyme capable of catalyzing the hydrolysis (cleavage) of bonds between nucleic acids within a DNA or RNA molecule, preferably a DNA molecule. According to the invention, the endonuclease is specifically an endonuclease variant. The endonuclease variants used in the present invention do not cleave the DNA or RNA molecule irrespective of its sequence, but recognize and cleave the DNA or RNA molecule at specific polynucleotide sequences, further referred to as "target sequences", "target sites", "recognition sites" or "recognition sequences". Target sequences recognized and cleaved by an endonuclease variant according to the invention are referred to as target sequences according to the invention.

The endonuclease variant can for example be a homing endonuclease (Paques et al. Curr Gen Ther. 2007 7:49-66), a chimeric Zinc-Finger nuclease (ZFN) resulting from the fusion of engineered zinc-finger domains with the catalytic domain of a restriction enzyme such as FokI (Porteus et al. Nat Biotechnol. 2005 23:967-973) or a chemical endonuclease (Arimondo et al. Mol Cell Biol. 2006 26:324-333; Simon et al. NAR 2008 36:3531-3538; Eisenschmidt et al. NAR 2005 33 :7039-7047; Cannata et al. PNAS 2008 105:9576-9581). For chemical endonucleases, a chemical or peptidic cleaver is conjugated either to a polymer of nucleic acids or to another DNA recognizing a specific target sequence, thereby targeting the cleavage activity to a specific sequence.

The endonuclease variant is preferably a homing endonuclease, also known as meganuclease (s). Such homing endonucleases are well-known to the art (see e.g. Stoddard, Quarterly Reviews of Biophysics, 2006, 38:49-95). Homing endonucleases recognize a DNA target sequence and generate a single- or double-strand break. Homing endonucleases are highly specific, recognizing DNA target sites ranging from 12 to 45 base pairs (bp) in length, usually ranging from 14 to 40 bp in length. The homing endonuclease variant according to the invention may for example correspond to a LAGLIDADG endonuclease, to a HNH endonuclease, or to a GIY-YIG endonuclease. Examples of such endonuclease include *I-Sce I, I-Chu I, I-Cre I, I-Csm I, PI-Sce I, PI-Tli I, PI-Mtu I, I-Ceu I, I-Sce II, I-Sce III, HO, PI-Civ I, PI-Ctr I, PI-Aae I, PI-Bsu I, PI-Dha I, PI-Dra I, PI-Mav I, PI-Mch I, PI-Mfu I, PI-Mfl I, PI-Mga I, PI-Mgo I, PI-Min I, PI-Mka I, PI-Mle I, PI-Mma I, PI-Msh I, PI-Msm I, PI-Mth I, PI-Mtu I, PI-Mxe I, PI-Npu I, PI-Pfu I, PI-Rma I, PI-Spb I, PI-Ssp I, PI-Fac I, PI-Mja I, PI-Pho I, PI-Tag I, PI-Thy I, PI-Tko I, PI-Tsp I, I-MsoI.*

In a preferred embodiment, the homing endonuclease variant is a LAGLIDADG endonuclease such as I*-SceI,* I*-CreI,* I*-CeuI,* I*-MsoI,* and I*-DmoI.*

In a most preferred embodiment, said LAGLIDADG endonuclease is I*-CreI.* Wild-type I-*CreI* is a homodimeric homing endonuclease that is capable of cleaving a 22 to 24 bp double-stranded target sequence. The sequence of a wild-type monomer of I*-CreI* includes the sequence shown as SEQ ID NO: 1 (which corresponds to the I*-CreI* sequence of pdb accession number 1g9y).

In the present patent application, the I-*Cre*I variants may comprise an additional alanine after the first methionine of the wild type I*-CreI* sequence, and three additional amino acid residues at the C-terminal extremity (see sequence of SEQ ID NO: 2). These three additional amino acid residues consist of two additional alanine residues and one aspartic acid residue after the final proline of the wild type I*-CreI* sequence. These additional residues do not affect the properties of the enzyme. For the sake of clarity, these additional residues do not affect the numbering of the residues in I*-CreI* or variants thereof. More specifically, the numbering used herein exclusively refers to the position of residues in the wild type I*-CreI* enzyme of SEQ ID NO: 1. For instance, the second residue of wild-type I*-CreI* is in fact the third residue of a variant of SEQ ID NO: 2 since this variant comprises an additional alanine after the first methionine.

In the present application, I*-CreI* variants may be homodimers (meganuclease comprising two identical monomers) or heterodimers (meganuclease comprising two non-identical monomers). It is understood that the scope of the present invention also encompasses the I-*Cre*I variants *per se,* including heterodimers (WO2006097854), obligate heterodimers (WO2008093249) and single chain meganucleases (WO03078619 and WO2009095793) as non limiting examples, able to cleave one of the sequence targets in the algal genome. The invention also encompasses hybrid variant *per se* composed of two monomers from different origins (WO03078619).

The endonuclease according to the present application is a "variant" endonuclease, i.e. an endonuclease that does not naturally exist in nature and that is obtained by genetic engineering or by random mutagenesis. The variant endonuclease according to the invention can for example be obtained by substitution of at least one residue in the amino acid sequence of a wild-type, endonuclease with a different amino acid. Said substitution(s) can for example be introduced by site-directed mutagenesis and/or by random mutagenesis. In the frame of the present invention, such variant endonucleases remain functional, i.e. they retain the capacity of recognizing and specifically cleaving a target sequence.

The variant endonuclease used in the invention cleaves a target sequence that is different from the target sequence of the corresponding wild-type endonuclease. For example, the target sequence of a variant I*-CreI* endonuclease is different from the sequence of SEQ ID NO:3 (palindromic sequence C1221 derived from the wild-type *IcreI* recognition site). Methods for obtaining such variant endonucleases with novel specificities are well-known in the art.

The present invention is based on the finding that such variant endonucleases with novel specificities can be used to allow efficient targeted genomic engineering within the algal genomes, thereby considerably increasing the usability of these organisms for various biotechnological applications.

In the frame of the present description, "algae" or "algae cells" or "cells", refer to different species of algae that can be used as hosts for genomic transformation using the meganucleases of the present description, polynucleotides and vectors encoding them.

According to the invention, the alga is specifically a diatom. For example the diatom is selected from *Amphora, Chaetoceros, Cyclotella, Cylindrotheca, Monoraphidium, Navicula, Nitzschia, Phaeodactylum,* and *Thalassiosira.*

By "gene" it is meant the basic unit of heredity, consisting of a segment of DNA arranged in a linear manner along a chromosome, which codes for a specific protein or segment of protein. A gene typically includes a promoter, a 5' untranslated region, one or more coding sequences (exons), optionally introns and a 3' untranslated region. The gene may further be comprised of terminators, enhancers and/or silencers.

By "genome" it is meant the entire genetic material contained in a cell such as nuclear genome, chloroplastic genome, mitochondrial genome...

By "nearest genes" it is meant the two genes that are located closest to the target sequence, centromeric and telomeric to the target sequence respectively.

As used herein, the term "locus" is the specific physical location of a DNA sequence (e.g. of a gene) on a chromosome. As used in this specification, the term "locus" usually refers to the specific physical location of an endonuclease's target sequence on a chromosome. Such a locus, which comprises a target sequence that is recognized and cleaved by an endonuclease according to the invention, is referred to as "locus according to the invention".

By "site of interest" it is meant a locus inside a genome containing an endonuclease target sequence or a putative endonuclease target sequence for an engineered endonuclease with a modified specificity such as said endonuclease is able to cleave said target inside said site of interest to achieve a targeted genomic event.

As used herein, the term "transgene" refers to a sequence inserted at a site of interest in an algal genome. Preferably, it refers to a sequence encoding a polypeptide. Preferably, the polypeptide encoded by the transgene is either not expressed, or expressed but not biologically active, in the algae or algal cells in which the transgene is inserted. Most preferably, the transgene encodes a polypeptide useful for increasing the usability and the commercial value of algae. Also, the transgene can be a sequence inserted at a site of interest in an algae genome for producing an interfering RNA.

As used herein, the expressions "gene of interest," "nucleotide sequence of interest", "nucleic acid of interest" or "sequence of interest" refer to any nucleotide or nucleic acid sequence that encodes a protein or other molecule that is desirable for expression in an algal cell (e.g.. for production of the protein or other biological molecule [e.g., an RNA product like interfering RNA as a non limiting example] in the target cell). The nucleotide sequence of interest is generally operatively linked to other sequences which are needed for its expression, e.g., a promoter. Further, the sequence itself may be regulatory in nature and thus of interest for expression in the target cell.

By "homologous" it is meant a sequence with enough identity to another one to lead to homologous recombination between sequences, more particularly having at least 95% identity, preferably 97% identity and more preferably 99%.

"Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting.

By "mutation" it is meant the substitution, deletion, insertion of one or more nucleotides/amino acids in a polynucleotide (cDNA, gene) or a polypeptide sequence. Said mutation can affect the coding sequence of a gene or its regulatory sequence. It may also affect the structure of the genomic sequence or the structure/stability of the encoded mRNA.

In a preferred embodiment, the present description discloses endonuclease variants to perform targeted gene knock-out in algae, more specifically in diatoms. Gene knock-out is the most powerful tool for determining gene function or permanently modifying the phenotypic characteristics of a cell. The repair of double strand DNA breaks (DSB) in mammalian cells occurs via the distinct mechanisms of homology directed repair (HDR) or NHEJ. Although HDR typically uses the sister chromatid of the damaged DNA as a template from which to perform perfect repair of the genetic lesion, NHEJ is an imperfect repair process that often results in changes to the DNA sequence at the site of the DSB. During NHEJ, the cleaved DNA is further resected by exonuclease activity, and more bases may be added in an imprecise manner before the two ends of the damaged DNA are rejoined. The subject-matter of the present invention is also a method for making a targeted knock-out in diatoms wherein a targeted double-stranded cleavage at a site of interest inside the algae genome is induced by an endonuclease variant, and repaired by the non-homologous end-joining pathway (NHEJ), resulting in loss of gene function. Preferably, the endonuclease of the present invention is a meganuclease.

In a particular aspect of this embodiment the knocked-out diatom is made by introducing into said diatom, an endonuclease as defined above, so as to induce a double stranded cleavage at a site of interest of the genome comprising a DNA recognition and cleavage site of said endonuclease, and thereby generate genetically modified diatoms knocked-out for the gene located at this site of interest of the genome, said modified diatoms having repaired the double-strands break by NHEJ, and isolating said genetically modified algae knocked-out by any appropriate means.

In another particular aspect of this embodiment the knocked-out algae is made by introducing into a diatom, 1) an endonuclease as defined above, so as to induce a double stranded cleavage at a site of interest of the genome comprising a DNA recognition and cleavage site of said endonuclease, 2) a knock-out template to be introduced is flanked by sequences sharing homologies with the region surrounding the genomic DNA cleavage site and thereby generates genetically modified algae knocked-out for the gene located at this site of interest of the genome, said modified algae having repaired the double-strands break by HDR, and isolating said genetically modified diatoms knocked-out by any appropriate means.

In another preferred embodiment, the present disclosure provides endonuclease variants to target sequence insertions (knock-in) into chosen loci of the genome. In this embodiment the knocked-in algae is made by introducing into a diatom , 1) an endonuclease as defined above, so as to induce a double stranded cleavage at a site of interest for sequence insertion in the genome comprising a DNA recognition and cleavage site of said endonuclease, 2) a knock-in template to be introduced flanked by sequences sharing homologies with the region surrounding the genomic DNA cleavage site and thereby generating genetically modified algae at this site of interest of the genome, said modified algae having repaired the double-strands break by HDR, and isolating said genetically modified diatoms by any appropriate means.

By "sequence insertion", it is intended the introduction into a target genome of an exogenous nucleotidic sequence.

By "targeting DNA construct/minimal repair matrix/repair matrix/template (knock-out template or knock-in template)" it is intended a DNA construct comprising a first and second portion of sequences which are homologous to regions 5' and 3' of the DNA target *in situ,* at a site of interest in the diatom genome. The DNA construct also comprises a third portion positioned between the first and second portion which can comprise some homology with the corresponding DNA sequence *in situ* (in the cases of allele/promoter swap as non-limiting examples) or alternatively can comprise no homology with the regions 5' and 3' of the DNA target *in situ* (insertion of a selectable marker). Following cleavage of the DNA target, a homologous recombination event is stimulated between the genome containing the targeted gene or part of the targeted gene and the repair matrix, wherein the genomic sequence containing the DNA target is replaced by the third portion of the repair matrix and a variable part of the first and second portions of the repair matrix. The repair matrix can also be endogenous such as a chromosomal sequence of interest. The chromosomal sequence of interest can be either located on the same chromosome as the genomic locus of interest, or on a different chromosome.

Preferably, homologous sequences of at least 50 bp, preferably more than 100 bp and more preferably more than 200 bp are used. Therefore, the targeting DNA construct is preferably from 200 bp to 6000 bp, more preferably from 1000 bp to 2000 bp. Indeed, shared DNA homologies are located in regions flanking upstream and downstream the site of the break and the DNA sequence to be introduced should be located between the two arms. The targeting construct comprises advantageously a positive selection marker between the two homology arms and eventually a negative selection marker upstream of the first homology arm or downstream of the second homology arm. The marker(s) allow(s) the selection of algae having inserted the sequence of interest by homologous recombination at the target site.

For the insertion of a sequence, DNA homologies are generally located in regions directly upstream and downstream to the site of the break (sequences immediately adjacent to the break; minimal repair matrix). However, when the insertion is associated with a deletion of ORF sequences flanking the cleavage site, shared DNA homologies are located in regions upstream and downstream the region of the deletion.

In a particular aspect of this embodiment, sequence insertions can be used to modify a targeted existing gene, by correction or replacement of said gene (allele swap as a non-limiting example), or to up or down regulate the expression of the targeted gene (promoter swap as non-limiting example), said targeted gene correction or replacement conferring one or several commercially desirable traits.

In another particular aspect of this embodiment, sequence insertions can be used to introduce new sequences or genes of interest increasing the potential exploitation of diatoms by conferring them commercially desirable traits for various biotechnological applications.

As non-limiting examples, traits that can be engineered in Algae and that are comprised in the scope of the present invention can be traits related to: - Quorum Sensing (QS) (QS allows cell-to-cell communication: sensing the environment. This system well-described in bacteria, uses a chemical signaling mechanism to coordinate expression of various genes when a sufficient population of bacteria has been reached. Several QS signaling molecules have been identified in bacteria, most notably the N-acylhomoserine lactone (AHL) family, and the Autoinducer 1 (acylated homoserine lactone) and Autoinducer 2 (a furanosyl borate diester) compounds (Teplitski et al., Plant Physiology, 2004). Interestingly, certain algae such as the red macroalgae *Delisea pulchra,* secrete GS mimic molecules (furanone compounds), that interfere with the bacterial AHL QS sensing signals, and thus can be used to prevent growth of harmful bacterial pathogens (Hentzer and Givskov, The Journal of Clinical Investigation, 2003; Williams, Microbiology, 2007). AHL mimic substances that activate QS have been studied in the model algal organism *Chlamydomonas reinhardtii,* which is amenable to genetic and molecular studies (the sequence is also available: http://www.biology.duke.edu/chlamy_genome/). The identification and targeting of these algal mimic compounds can be applied to the prevention of marine bacterial /algal biofilm development and algal blooms, and in the case of the algal furanones from marine algae, to create antipathogenic drugs (Kuehl et al., 2009, Antimicrobial Agents and Chemotherapy). Finally, certain toxic algae (such as *Gymnodinium catenatum*) living with marine bacteria (that release neurotoxins infecting shellfish and causing paralytic shellfish poisoning), require the QS signals from bacteria (sideopherones and borate) to know there is enough iron available, - Secretion of hydrocarbons (without limitation, lipids, isoprenoids, polyunsaturated aldehydes as source of alkanes or alkenes, production of polymers such as alginates), - Fatty acid composition (lipid branching), - Lipid accumulation (biofuel production). A target for increasing biofuel production has been isolated in the green algae Chlamydomonas, (Li et al; 2010, Metabolic Engineering). In this study, inhibition of starch synthesis in a starchless mutant within the gene encoding for ADP-glucose pyrophophorylase led to hyper-accumulation of fatty acids and triacylglycerol (TAG).- Lipids and antibacterial, therauptic applications: The polyunsaturated fatty acid from *P. tricornutum,* called eicosapentaenoic acid (EPA), was also recently shown to act as an effective anti-bacterial reagent against pathogenic bacteria such as the multi-drug resistant Staphyloccus aureus (MRSA), not susceptible to most known antibiotics (Desbois et al;, 2009, Mar Biotechnol), - Photosynthesis (additional pigments to enlarge useful light wavelengths), - Pigment production (Carotenoids and Phycobiliproteins as non-limiting examples), - Herbicide resistance, - Mercury volatilization, - Frustule composition and organization (nanostructured materials and devices). Diatoms display a diverse array of silicon structures at the nano- to millimeter scale and diatom nanotechnology can be applied to the fields of biophotonics, photoluminescence, microfluids, silica sequestering, multiscale porosity, silica sequestering of proteins, detection of trace gases, computer design and controlled drug delivery (Gordon et al; 2008, Trends in Biotechnology). The silica deposition vesicles (SDV), silicon transport vesicles, clathrin pits, and microtubules and silaffins (long chain polyamines) are major components of the silicon transport pathway, necessary for silica precipitation. - Biosafety issues, such as, in a non-limiting example, to avoid a transgene of interest to disseminate in natural ecosystems.

Some genetic elements that are related to said previous engineerable traits can be, without limitation : Acylhomoserine Lactone (AHL) (Jun Sum Kim et al Biotechnol and Bioprocess Engineering 2007), delta(12)-fatty acid dehydrogenase (fad2), fatty acid desaturase thioesterase (TE), Tla1 antennae. Similar acting genes, or antenna mutant (e.g. chlorophyll a oxygenase-CAO),(advantageous in high light only), Aldolase and TPI D- fructose 1,6-bisphosphatase / sedoheptulose 1,7-bisphosphatase, Blue Fluorescent protein, Overexpressed cystathione γ-synthase, Elevated dihydropicolinate synthase and suppressed lysine ketobutyrate reductase/saccharopine dehydrogenase. 5-enolpyruvylshikimate-3-phosphate synthase (EPSPS), Phytoene desaturase, glyphosate oxidoreductase, acetolactate synthase, Nitrilase, phosphinothricin N-acetyltransferase, 4-hydroxyphenyl-pyruvate-dioxygenase (HPPD), Protoporphyrinogen oxidase (PPO or protox), Glutamine synthetase, Helicase, replicase, viral coat protein, exo-alpha-sialidase (involved in N-glycan degradation and sphingolipid metabolism), silaffins and silicic acid transporters, furanones, PtCPF1 protein from P. tricornutum (and all members of the cryptochrome/photolyase family (CPF), Diatom Si transporters (SIT) (Thamatrakoln and Hildebrand, 2008, Plant Physiology), Proteins involved in manipulation of silicon (Mock et al; 2008, PNAS), FCP proteins (fucoxanthin chlorophyll a/c proteins), RuBisCo, Silaffins, Silaffin transporters, ADP-glucose pyrophosphorylase and EPA (eicosapentaenoic acid).

Other uses of this particular aspect of the invention, include the insertion of sequences that can be an interfering sequence i.e a sequence silencing genes of interest or respective products of said genes, per se (RNA interference process well-known in the art) or by the interfering agent coded by said interfering sequence, these interfering sequences conferring one or several commercially desirable traits by their silencing actions. These interfering sequences can be one or more sequences selected from siRNAs, shRNAs, miRNAs, cDNAs.

As mentioned above the term "interfering sequence" refers to a sequence able to silence a gene per se or by the "interfering agent" encoded by this interfering sequence. As a non-limiting example, said interfering sequence can code for a protein inhibitor i.e the interfering agent in this case, this protein inhibitor being able to interact with and inhibit a targeted enzyme, this silencing process conferring to the algae host a commercially advantageous trait.

Gene silencing by RNAi has been characterized and used as a tool to generate targeted gene knockdown or knockout mutants in Phaeodactylum tricornutum (De Riso, V. et al 2009). However, as previously used this RNAi approach cannot be used to create strains containing stable gene knock-down or knock-outs. Use of meganucleases according to the present invention is ideally suited for this.

Interfering RNAs (iRNAs) include, miRNAs, siRNAs and shRNAs; an interfering RNA is also an interfering agent as described above.

As shown at least in mammalian cells, the enzyme Dicer cleaves long dsRNAs into short-interfering RNAs (siRNAs) of approximately 21-23 nucleotides. One of the two siRNA strands is then incorporated into an RNA-induced silencing complex (RISC). RISC compares these "guide RNAs" to RNAs in the cell and efficiently cleaves target RNAs containing sequences that are perfectly, or nearly perfectly complementary to the guide RNA. "iRNA construct" also includes nucleic acid preparation designed to achieve a RNA interference effect, such as expression vectors able of giving rise to transcripts which form dsRNAs or hairpin RNA in cells, and or transcripts which can produce siRNAs *in vivo.*

A "short interfering RNA" or "siRNA" comprises a RNA duplex (double-stranded region) and can further comprises one or two single-stranded overhangs, 3' or 5' overhangs. Each molecule of the duplex can comprise between 17 and 29 nucleotides, including 17, 18, 19, 20, 21, 22, 23, 24, 25, 26, 27, 28, and 29 nucleotides. siRNAs can additionally be chemically modified.

"MicroRNAs" or "miRNAs" are endogenously encoded RNAs that are about 22-nucleotide-long, that post-transcriptionally regulate target genes and are generally expressed in a highly tissue-specific or developmental-stage-specific fashion. At least more than 200 distinct miRNAs have been identified in plants and animals. These small regulatory RNAs are believed to serve important biological functions by two predominant modes of action: (1) by repressing the translation of target mRNAs, and (2) through RNA interference, that means cleavage and degradation of mRNAs. In this latter case, miRNAs function analogously to siRNAs. miRNAs are first transcribed as part of a long, largely single-stranded primary transcript (pri-miRNA) [Lee et al., 2002, EMBO J. 21: 4663-4670]. This pri-miRNA transcript is generally and possibly invariably, synthetized by RNA polymerase II and therefore is polyadenylated and may be spliced. It contains an approximate 80-nucleotides long hairpin structure that encodes for the mature, approximately 22-nucleotides miRNA part of one arm of the stem. In animal cells, this primary transcript is cleaved by a nuclear RNaseIII-type enzyme called Drosha (Lee et al, 2003, Nature 425:415-419) to liberate a hairpin mRNA precursor, or pre-miRNA of about-65 nucleotides long. This pre-miRNA is

then exported to the cytoplasm by exportin-5 and the GTP-bound form of the Ran cofactor (Yi et al, 2003, Genes and Development 17:3011-3016). Once in the cytoplasm, the pre-miRNA is further processed by Dicer, another RNaseIII enzyme to produce a duplex of about-22 nucleotides base pairs long that is structurally identical to a siRNA duplex (Hutvagner et al, 2001, Science 293:834-838). The binding of protein components of the RISC, or RISC cofactors, to the duplex results in incorporation of the mature, single-stranded miRNA into a RISC or RISC-like protein complex, while the other strand of the duplex is degraded (Bartel et al, 2004, Cell 116: 281-297). Thus, one can design and express artificial miRNAs based on the features of existing miRNA genes. The miR-30 (microRNA 30) architecture can be used to express miRNAs (or siRNAs) from RNA polymerase II promoter-based expression plasmids (Zeng et al, Methods enzymol. 392:371-380). In some instances the precursor miRNA molecules may include more than one stem-loop structure. The multiple stem-loop structures may be linked to one another through a linker, such as, for example, a nucleic acid linker, a miRNA flanking sequence, other molecules, or some combination thereof.

A "short hairpin RNA (shRNA)" refers to a segment of RNA that is complementary to a portion of a target gene (complementary to one or more transcripts of a target gene), and has a stem-loop (hairpin) structure, and which can be used to silence gene expression. A "stem-loop structure" refers to a nucleic acid having a secondary structure that includes a region of nucleotides which are known or predicted to form a double strand (stem portion) that is linked on one side by a region of predominantly single-stranded nucleotides (loop portion). The term "hairpin" is also used herein to refer to stem-loop structures.

In another particular aspect, the present description discloses endonuclease variants to insert genes at targeted chosen loci in algal genomes that can be considered as "safe harbors" for gene addition i.e. a loci allowing safe and stable expression of a transgene. The present description is based on the finding that such variant endonucleases with novel specificities can be used for inserting a gene into a "safe harbor" locus of the genome of a algae. In a preferred aspect, the locus further allows stable expression of the transgene. In another preferred aspect, the target sequence is only present once within the genome of said algae. Ideally, insertion into a good safe harbor locus should have no impact on the expression of other genes. In a preferred aspect a locus for targeted insertion is chosen close to a gene that is essential for survival of the targeted algae, said transgene inserted at this locus becoming genetically linked to this essential gene and the probability of their independent segregation from each other becoming extremely low. In this preferred aspect said gene essential for the survival of the targeted algae is a housekeeping gene. In a non-limiting list, housekeeping genes that are comprised in the scope of the present invention are those required for the maintenance of basal cellular function, like as non-limiting examples, transcription factors, translation factors (tRNA synthetases, RNA binding proteins), ribosomal proteins, RNA polymerases, processing proteins, Heat Shock Proteins, histones, cell cycle genes, metabolism genes (carbohydrate metabolism, citric acid cycle, lipid of fatty acid metabolism, amino acid metabolism, nitrogen metabolism, urea cycle, polyamine biosynthesis pathway, nucleotide synthesis), structural genes (cytoskeleton, organelle synthesis), carbon fixation, cell wall synthesis pathway and clathrin-mediated endocytosis.

Testing these properties is a multi-step process, and a first pre-screening of candidate safe harbor loci by bioinformatic means is desirable. One can thus first identify loci in which targeted insertion is unlikely to result in insertional mutagenesis.

In addition, in another specific embodiment, insertion of a genetic element into said locus does not substantially modify the phenotype of said diatoms (except for the phenotype due to expression of the genetic element). By "phenotype" it is meant an algae's or a algae cell's observable traits. The phenotype includes viability, growth, resistance or sensitivity to various marker genes, environmental and chemical signals, etc...

Once such a safe harbor locus has been selected, one can then construct a variant endonuclease specifically recognizing and cleaving a target sequence located within said locus. Alternatively, once a safe harbor locus has been selected, the skilled in the art can insert therein a target sequence that is recognized and cleaved by a known variant endonuclease.

Therefore, the description discloses a method for obtaining an endonuclease suitable for safely inserting a transgene into the genome of an algae for example without substantially modifying (i) expression of the nearest genes, and/or (ii) the cellular proliferation and/or the growth rate of the cell, tissue or individual.

The present description also discloses endonuclease variants to induce single-stranded annealing (SSA). When tandemly repeated homologous sequences surround a DSB, an efficient mode of DSB repair can be intra-chromosomal recombination by SSA between the two directly repeated homologous sequences, leading to the physical elimination of one repeat and all the sequences between the repeats. SSA is a powerful approach to excise sequences from the chromosome.

Site-specific recombinases such as Cre-lox and Flp recombinase systems have also been widely used in many cell types. Although these systems are efficient to perform marker removal, for example, their big drawback is that the final recombination event contains the exogenous or foreign recombination target site (typically a loxP site) which is not desirable in terms of Genetically Modified Organisms (GMO) issues, and remains functional, impeding future re-use of the same system in the cell. In addition, this exogenous footprint can lead to genomic instabilities and further chromosomal rearrangements.

Endonuclease-induced SSA-based excision, in contrast, efficiently leads to removal of, for example, marker sequences, leading to stable and precise recombination correction events, without leaving behind exogenous sequences. In other words, if one introduces a marker, with short regions of flanking homology, these sequences can then be later removed, leaving only the native wild type sequence, without any "scar" on the genome. This occurs by the highly efficient SSA recombination pathway. Marker removal by endonuclease induced SSA provides a major advantage in terms of generating non-GM strains or species. In addition, the marker can be repeatedly re-used again, which is an important issue in diatoms, since they lack a variety of different selection markers.

Only few publications refer to selection markers usable in Diatoms. Dunahay et al 1995 and Zaslavskaia et al 2001 report the use of the neomycin phosphotransferase II (nptII), that inactivates G418 by phosphorylation, in Cyclotella cryptic, Navicula saprophila and Phaeodactylum tricornutum species. Falciatore et al 1999, Fischer et al 1999 and Zaslavskaia et al 2001 report the use of the Zeocin resistance gene (Sh ble), acting by stochiometric binding, in Phaeodactylum tricornutum and Cylindrotheca fusiformis species. In Zaslavskaia et al 2001, the use of N-acetyltransferase 1 gene (Natl) conferring the resistance to Nourseothricin by enzymatic acetylation is reported in Phaeodactylum tricornutum and Thalassiosira pseudonana. It is understood that use of the previous specific selectable markers are comprised in the scope of the present invention and that use of other genes encoding other selectable markers including, for example and without limitation, genes that participate in antibiotic resistance are also comprised in the scope of the present invention.

Marker removal by the use of meganuclease provides a major advantage in terms of generating non-GM strains or species or by the fact that the few positive selection markers available in algae can be repeatedly used.

In another preferred embodiment, endonuclease variants described herein allow "transgene stacking", i.e the insertion of multiple transgenes into the same, chosen, locus in the genome of an algae, more specifically a diatom. Such targeted locations are referred to as "landing pads" in safe places within the genome. Endonuclease variants used in the present invention allow flexible consecutive and reproducible sequence insertion into the same locus of any species of algae. In a particular aspect of this preferred embodiment, endonuclease variants allow the link of multiple traits/genes in a recipient genome, i.e different sequences, alleles or traits, identified in separate algae strains or isolates, can be precisely re-introduced within a single industrial strain, without the need for sexual crossing.

In another preferred embodiment, endonuclease variants described herein allow "pathway engineering". Since endonucleases described herein allow a broad range of genomic modifications (allele swap, gene stacking, promoter swap, gene knock-in or knock-out, inducible sequence pop-out as non-limiting examples), metabolic pathway engineering, increasing the usability and the commercial value of algae, is comprised in the scope of the present description.

In another preferred embodiment, endonuclease variants provided in the present disclosure target sequences selected from the group consisting of SEQ ID NO 20 to 39 from the genome of *Phaeodactylum tricornutum,* and SEQ ID NO 40 to 58 from the genome of *Thalassiosira pseudonana.*

The subject-matter of the present description is also a polynucleotide fragment encoding a variant of an endonuclease as defined above. Preferably, the subject-matter of the present description is also a polynucleotide fragment encoding a variant meganuclease as defined above; said polynucleotide may encode for instance one monomer of a homodimeric or heterodimeric variant, or two domains/monomers of a single-chain meganuclease or any variants as defined above. It is understood that the subject-matter of the present description is also a polynucleotide fragment encoding one of the variant species as defined above, obtained by any well-known method in the art.

The subject-matter of the present description is also a recombinant vector for the expression of an endonuclease variant as defined above. The subject-matter of the present description is also a recombinant vector for the expression of any variant according to the invention. The recombinant vector comprises at least one polynucleotide fragment encoding any as defined above.

By "vector" is intended to mean a nucleic acid molecule capable of transporting another nucleic acid to which it has been linked. A vector which can be used in the present invention includes, but is not limited to, a viral vector, a plasmid, a RNA vector or a linear or circular DNA or RNA molecule which may consists of a chromosomal, non chromosomal, semisynthetic or synthetic nucleic acids. Preferred vectors are those capable of autonomous replication (episomal vector) and/or expression of nucleic acids to which they are linked (expression vectors). Large numbers of suitable vectors are known to those skilled in the art and commercially available. Some useful vectors include, for example without limitation, pGEM13z. pGEMT and pGEMTEasy {Promega, Madison, WI); pSTBluel (EMD Chemicals Inc. San Diego, CA); and pcDNA3.1, pCR4- TOPO, pCR-TOPO-II, pCRBlunt-II-TOPO (Invitrogen, Carlsbad, CA).

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; *TRP1, URA3* and *LEU2* for *S. cerevisiae*; tetracycline, rifampicin or ampicillin resistance in *E. coli.* Preferably said vectors are expression vectors, wherein the sequence(s) encoding the variant/single-chain meganuclease of the invention is placed under control of appropriate transcriptional and translational control elements to permit production or synthesis of said variant. Therefore, said polynucleotide is comprised in an expression cassette. More particularly, the vector comprises a replication origin, a promoter operatively linked to said polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is a heterodimer, the two polynucleotides encoding each of the monomers are included in one vector which is able to drive the expression of both polynucleotides, simultaneously. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-β-D-thiogalacto-pyranoside (IPTG) and eukaryotic heat shock promoter which is induced by increased temperature.

In some embodiments, the vector for the expression of the endonucleases described herein can be operably linked to an algal-specific promoter. In some embodiments, the algal-specific promoter is an inducible promoter. In some embodiments, the algal-specific promoter is a constitutive promoter. Promoters that can be used include, for example without limitation, a Pptca1 promoter (the CO2 responsive promoter of the chloroplastic carbonic anyhydrase gene, ptca1, from *P. tricornutum*), a NIT1 promoter, an AMT1 promoter, an AMT2 promoter, an AMT4 promoter, a RH1 promoter, a cauliflower mosaic virus 35S promoter, a tobacco mosaic virus promoter, a simian virus 40 promoter, a ubiquitin promoter, a PBCV-I VP54 promoter, or functional fragments thereof, or any other suitable promoter sequence known to those skilled in the art.

In another most preferred embodiment the vector is a shuttle vector, which can both propagate in E. coli (the construct containing an appropriate selectable marker and origin of replication) and be compatible for propagation or integration in the genome of the selected algae.

According to another advantageous embodiment of said vector, it includes a targeting construct comprising sequences sharing homologies with the region surrounding the targeted genomic DNA cleavage site in algae as defined above.

For instance, said sequence sharing homologies with the regions surrounding the genomic DNA cleavage site of the variant is a fragment of the targeted genomic DNA. Alternatively, the vector encoding for an endonuclease variant/single-chain meganuclease and the vector comprising the targeting construct are different vectors.

Endonucleases provided in the present invention can be delivered in various formats: DNA, messenger RNA, or even as a protein.

A variety of different methods are known for the introduction of DNA into host cell nuclei. In various embodiments, the vectors can be introduced into algae nuclei by, for example without limitation, electroporation, particle inflow gun bombardment, or magnetophoresis. In various embodiments, the transformation methods can be coupled with one or more methods for visualization or quantification of nucleic acid introduction to one or more algae.

Direct microinjection of purified endonucleases in algae can be considered. Also appropriate mixtures commercially available for protein transfection can be used to introduce endonucleases in algae according to the present invention. More broadly, any means known in the art to allow delivery inside cells or subcellular compartments of agents/chemicals and molecules (proteins) can be used to introduce endonucleases in algae including liposomal delivery means, polymeric carriers, chemical carriers, lipoplexes, polyplexes, dendrimers, nanoparticles, emulsion, natural endocytosis or phagocytose pathway as non-limiting examples.

The subject matter of the present description is also a kit for making knock-out/knock-in in algae comprising at least an endonuclease and/or one expression vector, as defined above. Preferably, the subject matter of the present description is also a kit for making knock-out/knock-in in algae comprising at least a meganuclease and/or one expression vector, as defined above. More preferably, the kit further comprises a targeting DNA comprising a sequence that inactivates the targeted gene flanked by sequences sharing homologies with the region of the targeted gene surrounding the DNA cleavage site of said meganuclease. In addition, for making knocked-in algae, the kit includes also a vector comprising a sequence of interest to be introduced in the genome of said algae and eventually a selectable marker gene, as defined above.

In accordance with some embodiments of the present description, and combinations between these embodiments, bioprocess algae containing commercially desirable traits by the use of one or more endonuclease variants as described herein, are comprised in the scope of the present description. Particularly, is comprised in the scope of the present description a targeted genome engineered algae (i.e an algae whose genome has been modified at a targeted site of interest) wherein said algae genome contains at least one gene modified by one or more endonuclease variants as described herein. More particularly, is comprised in the scope of the present description a targeted genome engineered algae encoding at least one gene conferring an advantageous trait for biotechnological applications, selected from the group of genes encoding quorum sensing, secretion of hydrocarbons, fatty acid composition, lipids accumulation, enhanced photosynthesis, pigments production, mercury volatilization, frustule composition or organization, mitigation genes in a non-exhaustive list, wherein said at least one gene has been introduced by one or more endonuclease variants as described herein.

The above written description of the invention provides a manner and process of making and using it such that any person skilled in this art is enabled to make and use the same, this enablement being provided in particular for the subject matter of the appended claims, which make up a part of the original description.

As used above, the phrases "selected from the group consisting of', "chosen from" and the like include mixtures of the specified materials.

Where a numerical limit or range is stated herein, the endpoints are included. Also, all values and sub-ranges within a numerical limit or range are specifically included as if explicitly written out.

The above description is presented to enable a person skilled in the art to make and use the invention, and is provided in the context of a particular application and its requirements.

Having generally described this invention, a further understanding can be obtained by reference to certain specific examples, which are provided herein for purposes of illustration only, and are not intended to be limiting unless otherwise specified.

### EXAMPLES

Microalgae are considered as one of the best alternatives to produce advanced liquid fuels such as biodiesel and bio-jet fuels, due to their capacity to synthesize large quantities of triacylglcerols (TAG). The 'microalgae for fuel' concept has been explored over the last 12 years, but previous attemps to increase lipid production by focusing strictly on fatty acid biosynthesis (Dunahay et al. 1996, ; Marillia et al. 2003; Roesler et al. 1997), increasing the availability of glycerol (Vigeolas et al; 2007) or over-expression of genes in the TAG biosynthesis pathway (Bouvier-Nave et al. 2000; Jako et al. 2001; Weselake et al. 2008; Zheng et al; 2008; Zou et al. 1997) have all shown limited success.

In green microalgae, starch synthesis shares common carbon precursors with lipid synthesis. STA6 codes for the catalytic small sub-unit of ADP-glucose pyrophosphorylase (AGPase), which is a well-conserved protein within all algal and photosynthetic organisms and is necessary for ADP-glucose synthesis (Zabawinski et al., J. of Bacteriology, 2001). Furthermore, mutants within this locus fail to accumulate starch.

Recent evidence in the model microalga organism, *Chlamydomonas reinhardtii,* shows that shunting of photosynthetic carbon precursors from starch to TAG synthesis, by inactivation of STA6, results in a 10-fold increase of fatty acid and TAG lipids (Li et al; Metabolic Engineering, 2010; Li et al; Biotechnology and Bioengineering, 2010). Therefore, STA6 is an important target for understanding how carbon partioning, through the inactivation of starch, may increase lipid production, ideally without compromising viability and growth rates.

Therefore, a first main aspect of this invention concerns the engineering of meganucleases targeting the STA6 gene to create a stable loss of gene function. Another aspect of this invention uses meganucleases for targeted knock-in of constructs containing site-specific substitutions within STA6 to create precise disruptions (i.e. a 1 or 2 bp nucleotide substitution), which should define new separation of function alleles, that is mutations that specifically increase lipid production significantly without compromising viability, growth or yield. It can also be useful to add marker constructs either within or just in *cis* to the STA6 locus, so that the strain containing the mutated locus can be easily followed and maintained upon sexual crosses or during other molecular and genetic manipulations. A second main aspect of this invention illustrated in Example 2 addresses how meganucleases can target entire genomes, including intergenic regions to be used as landing pads for targeted insertions of interesting target constructs.

### Example 1 (for comparative purposes only): Engineering meganucleases targeting the STA6 gene in Chlamydomonas reinhardtii

### A) Disruption (Knock-Out) of the STA6 gene using meganucleases targeting the STA6 gene without a repair matrix.

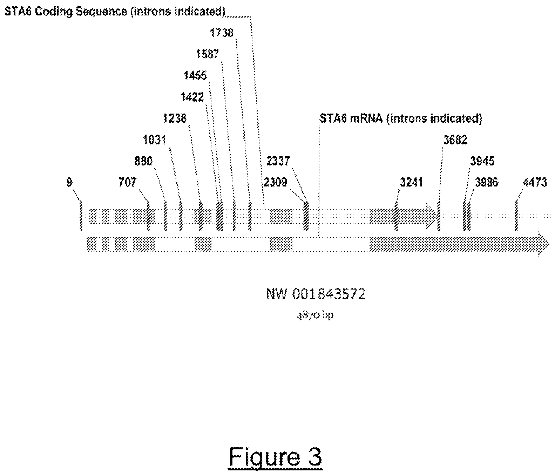

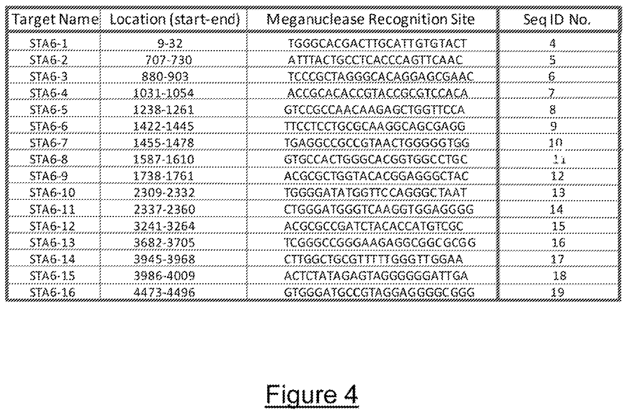

One strategy envisioned to knock-out the STA6 gene, involves mutating the coding sequence by non homologous end joining (NHEJ), using a STA6 meganuclease targeting a sequence within the open reading frame (see Figures 3 and 4 and Table1 below). In this case, the STA6 meganucleases recognizing exons 4 (STA6-2), 5 (STA6-5) or 7 (STA6-12) can be used to create a double-strand break in these exonic sites. In the absence of homology or a repair matrix, the double-stranded ends will either rejoin perfectly, or imperfectly, using micro-homologies near the break site. Imperfect end-joining, gives rise to small deletions or insertions within the open reading frame, and therefore generates loss of gene function. Lipid content in sta6 mutant strains compared to wild-type strains can be measured by testing the ability to produce TAG under both favorable (low light and nitrogen replete) and stress conditions that induce TAG synthesis (high-light and nitrogen-starved) (Hu et al., 2008, Plant J.). Using degenerate primers, homeologous to this sequence, one can easily identify, clone, and sequence STA6 homologs in other algal species and identify meganucleases to perform the same type of knock-out approach.

**Table 1 : Sequences and locations of targeted sites in the STA6 gene from Chlamydomonas reinhardtii (Gene bank accession number : NW001843572).**

| Target Name | Location (start-end) | Meganuclease Recognition Site | Seq ID No. |
|---|---|---|---|
| STA6-1 | 9-32 | TGGGCACGACTTGCATTGTGTACT | 4 |
| STA6-2 | 707-730 | ATTTACTGCCTCACCCAGTTCAAC | 5 |
| STA6-3 | 880-903 | TCCCGCTAGGGCACAGGAGCGAAC | 6 |
| STA6-4 | 1031-1054 | ACCGCACACCGTACCGCGTCCACA | 7 |
| STA6-5 | 1238-1261 | GTCCGCCAACAAGAGCTGGTTCCA | 8 |
| STA6-6 | 1422-1445 | TTCCTCCTGCGCAAGGCAGCGAGG | 9 |
| STA6-7 | 1455-1478 | TGAGGCCGCCGTAACTGGGGGTGG | 10 |
| STA6-8 | 1587-1610 | GTGCCACTGGGCACGGTGGCCTGC | 11 |
| STA6-9 | 1738-1761 | ACGCGCTGGTACACGGAGGGCTAC | 12 |
| STA6-10 | 2309-2332 | TGGGGATATGGTTCCAGGGCTAAT | 13 |
| STA6-11 | 2337-2360 | CTGGGATGGGTCAAGGTGGAGGGG | 14 |
| STA6-12 | 3241-3264 | ACGCGCCGATCTACACCATGTCGC | 15 |
| STA6-13 | 3682-3705 | TCGGGCCGGGAAGAGGCGGCGCGG | 16 |
| STA6-14 | 3945-3968 | CTTGGCTGCGTTTTTGGGTTGGAA | 17 |
| STA6-15 | 3986-4009 | ACTCTATAGAGTAGGGGGGATTGA | 18 |
| STA6-16 | 4473-4496 | GTGGGATGCCGTAGGAGGGGCGGG | 19 |

### B) Complete disruption (Knock-Out) and Gene Replacements (Knock-in) to create new alleles within the STA6 gene or promoter (using meganucleases and a KO/substitution repair matrix).

A second strategy to generate a loss of gene function, takes advantage of a knock-out repair matrix. This consists of disrupting a large region or even the entire STA6 open reading frame, using the two STA6 meganucleases STA6-1 and STA6-13 that respectively target just upstream and downstream of the open-reading frame and a knock-out repair matrix to generate a large deletion or disruption. To create a complete deletion with no insertion, the repair matrix is designed using sequences of flanking homology (typically 500 -1000 bp are used) outside and/or just within the STA6 open reading frame and deleted for the coding exon regions. One can also design the repair knock-out construct, with for example, a marker resistance gene such as the phytoene desaturase gene, (Frommolt et al. 2008, Mol. Biol. Evol.; Junchao et al., 2008, J. of Phycology), embedded between the same flanking homologous sequences for targeted gene replacement.

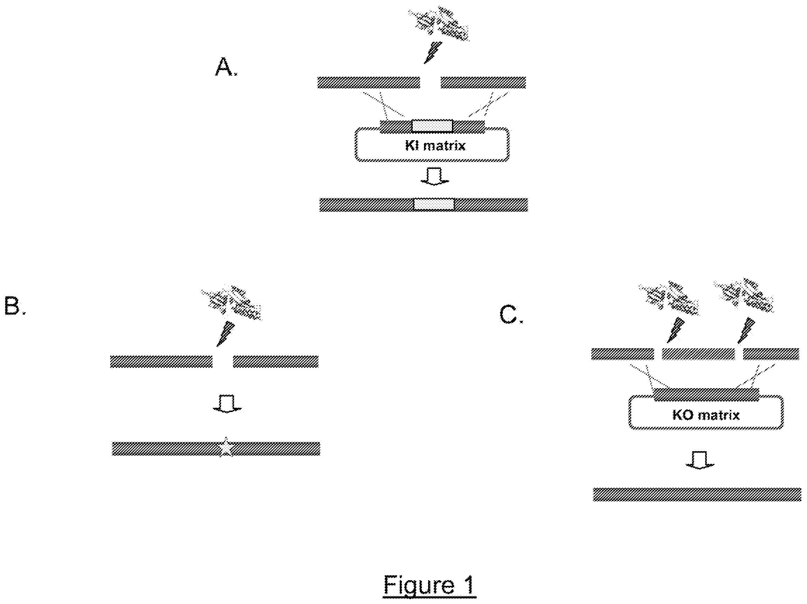

More subtle gene targeted replacements can be designed, using one or several of the meganucleases listed in Figure 1 and repair matrixes that contain specific DNA substitutions, or even randomly mutagenized sequences flanked by perfect homology to the STA6 locus. Site-specific substitutions within the STA6 locus may result in mutants with very different phenotypes, in terms of lipid production, growth and viability, as compared to a full deletion (null) construct. One can also modify gene expression, using the meganuclease targeting the promoter region (STA6-1), and a repair matrix containing a strong constitutive promoter, an attenuated version of the same endogenous promoter, or an inducible promoter, such as the heat-shock-inducible promoter hsp70, from the green alga as characterized in Chlamydomonas and Volvox (Cheng, et al., 2006, Gene, pp. 112-120).

### Example 2: Engineering meganucleases to target different sites in the genome of the diatom Phaeodactylum tricornutum for targeted integration (use of intergenic regions as safe landing pads for insertions constructs).

Example 1 provided an illustration of how to genetically modify and create various types of disruptions and substitutions within one defined locus, using meganucleases. With the recent advent of entire genome sequencing projects, meganucleases can be engineered to target sequences not only locally but globally. In this respect, target sites within intergenic regions, can be used as safe harbors or landing pads for insertion of interesting target sequences and constructs. In another words, these intergenic regions can be considered as safe because they should not perturb other neighbouring chromosomal genes either by disruption or by modifying their expression.

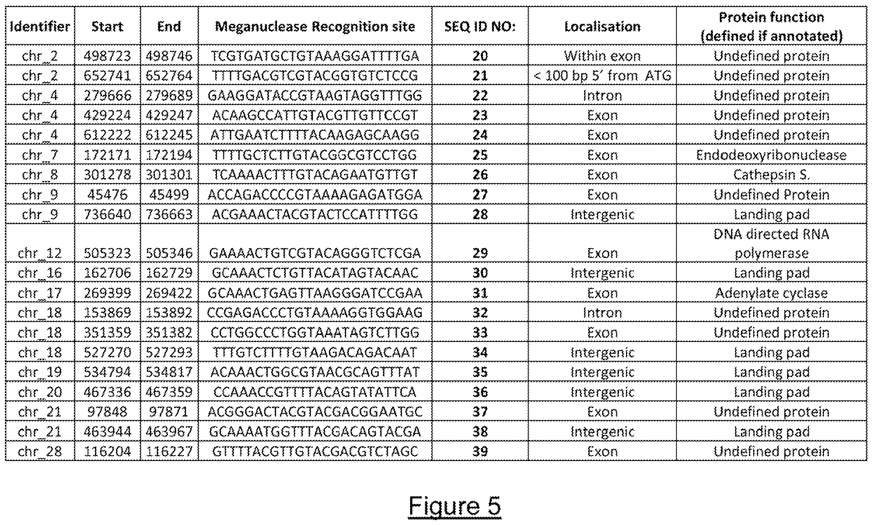

In Figure 5, different meganucleases cleaving sites within genes or intergenic regions located in the *P. tricornutum* genome have been identified. The six intergenic sites identified can be used as sites for insertion of marker constructs, RNA silencing constructs, or other sequences of interest.

Recently the fatty acid eicosapentaenic acid (EPA) from *Phaeodactylum tricornutum* was shown to display antibacterial activity in vitro against the pathogenic multidrug-resistant *Staphyloccus aureus* (MRSA), not susceptible to most antibiotics (Desbois, et al., 2009, Mar Biotechnol, pp. 45-52). In addition, EPA from *P. tricornutum* is able to inhibit the growth of the fish and shellfish pathogen *Listonella anguillarum,* arguing that overexpression of EPA could be useful in controlling disease in the mariculture industry and for human health purposes, when conventional antibiotics are not suitable. Studies involving EPA in this Diatom have only been performed at a biochemical level using purified extracts of this molecule. For the moment, many putative genomic EPA targets exist in *P. tricornutum,* but thus far none have been cloned, isolated and targeted for genomic modification. As EPA is difficult to extract and purify, it would be interesting to create EPA cDNA constructs either from algal species, plants or mammals, and insert such constructs using meganucleases at intergenic, safe site, as indicated in Figure 5. In this way, algae can serve as a tool for understanding EPA function, and perhaps to overexpress this molecule for downstream therapeutic uses.

### Example 3: Engineering meganucleases to target different sites in the genome of the diatom Thalassiosira pseudonana for targeted integration (use of intergenic regions as safe landing pads for insertions constructs).

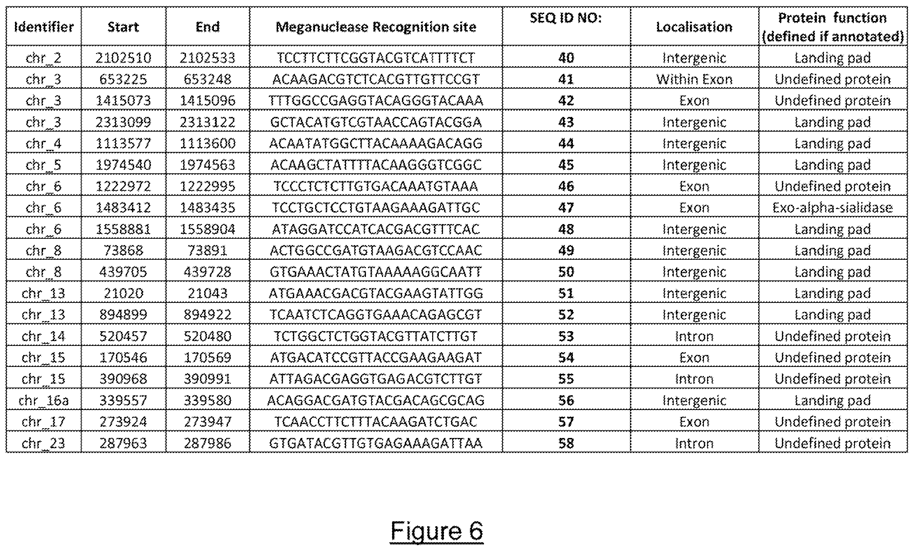

In Figure 6, different meganucleases cleaving sites within genes or intergenic regions located in the *Thalassiosira pseudonana* genome have been identified. The ten intergenic sites identified can be used as sites for insertion of marker constructs, RNA silencing constructs, or other sequences of interest.

### Example 4 (for comparative purposes only): Engineering meganucleases to target different sites in the genome of Chlorella (NC64A) for targeted integration (use of intergenic regions as safe landing pads for insertions constructs).

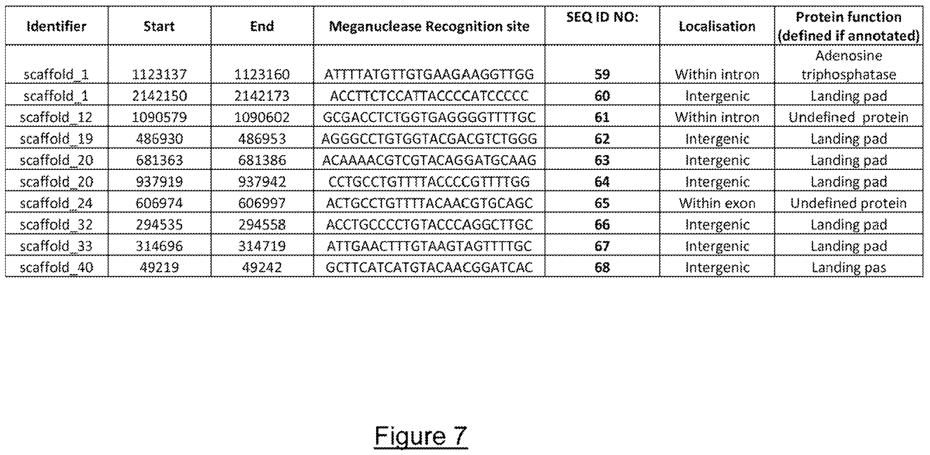

In Figure 7, different meganucleases meganucleases cleaving sites within genes or intergenic regions located in the *Chlorella (NC64A)* genome have been identified. The seven intergenic sites identified can be used as sites for insertion of marker constructs, RNA silencing constructs, or other sequences of interest.

### Example 5: Targeted genomic events in Thalassiosira pseudonana using TP7 engineered meganucleases.

### 1) Subcloning of TP7 meganuclease and nourseothricin acetyl transferase gene (natl) and open reading frames into diatom specific expression plasmids

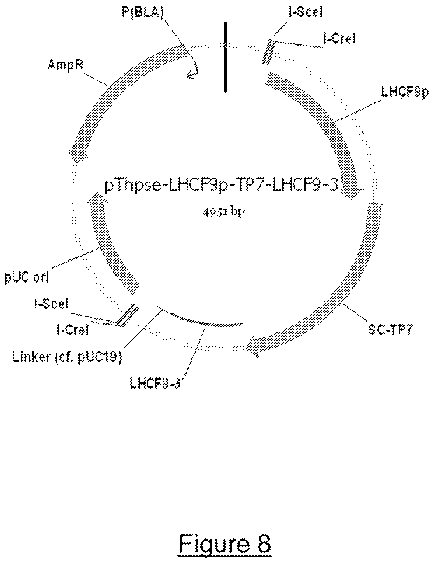

Meganuclease TP7 (TP7) targeting TP07.1 target (SEQ ID NO: 42 in figure 6) was obtained according to previously published methods (Grizot et al. 2009) and as described in the legend of figure 2. The TP7 single chain ORF (SEQ ID NO: 69) was excised by digestion from the plasmid pCLS7126 (SEQ ID NO: 70) then subcloned by ligation into a diatom specific expression plasmid. The latter contains regulatory regions (a LHCF9p promoter and a LHCF9-3' terminator) previously cloned into a vector containing only ampicillin resistance cassette and bacterial replication origin. The theoretical map of the resulting plasmid is depicted in figure 8 while its complete sequence is listed in SEQ ID NO: 71.

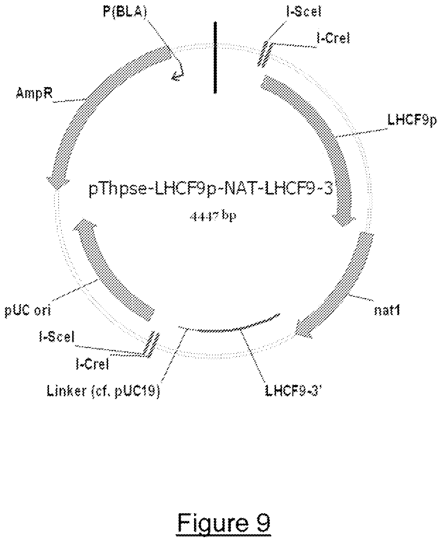

*nat*1 gene was subcloned into the same diatom specific expression vector between the promoter and the terminator regions. The theoretical map of the resulting plasmid is depicted in figure 9 while its complete sequence is listed in SEQ ID NO: 72.

### 2) Diatom culture CCMP1335 transformation

Diatom culture CCMP1335, species *Thalassiosira pseudonana* was genetically transformed by Cytopulse electroporation technology (Cellectis SA): 10⁷ cells were collected from an exponentially growing culture (concentration not exceeding 10⁶ cells·ml⁻¹) by centrifugation at 2500 rpm for 15 minutes. The supernatant was discarded and the pellet resuspended in 200 µl electroporation buffer to which 3 µg of TP7 expression plasmid (figure 8, SEQ ID NO: 71) and 3 µg of nat plasmid (figure 9, SEQ ID NO: 72) were previously added. The mix was then transferred to prechilled BioRad electroporation cuvettes (0.4 cm gap). Electroporation was performed in a CytoLVT-S (Cellectis Inc.) using the following program:
4 X (1200 V 0.2 ms) 50 ms interval 8 X (800 V 0.8 ms)
Group 1 pulses are separated by a 0.2 ms gap. Group 2 pulses are separated by a 2 ms gap.

After electroporation, cuvettes were put back on ice until next step. Electroporated cells were diluted into 2 mL complete growth medium (40 g Sigma Sea Salts in 980 mL sterile MilliQ water + 20 ml Sigma F2 enrichment solution), were transferred into a plate well or a 25 cm² flask and were incubated overnight in a growth chamber.

About 5x10⁶ cells are plated onto agar plates filled with 25 mL of solid medium (20 g Sigma Sea Salts in 980 mL sterile MilliQ water + 20 ml Sigma F2 enrichment solution + 10 g Pure Agar + 100 mg nourseothricin).

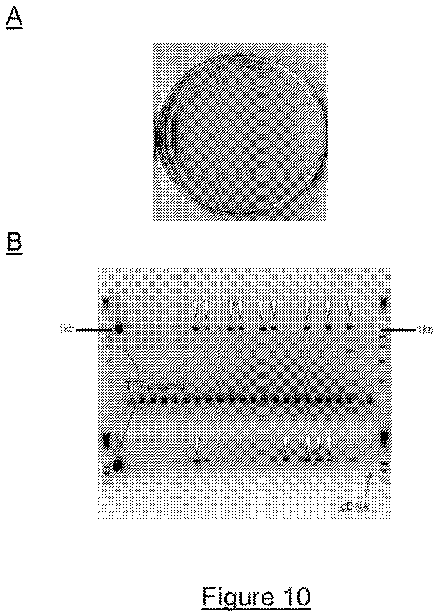

About 400 nourseothricin resistant colonies were recorded upon selection (figure 10A). 44 of them were PCR screened for the presence of TP7 meganuclease into the diatom genome (figure 10B). Fourteen strains (arrowheads) were transferred to liquid in order to increase algal biomass and isolate, DNA, RNA, and proteins for deep sequencing experiments, meganuclease expression, meganuclease protein accumulation into the cells.

### 3) DNA. RNA and protein isolation

DNA was isolated 41 days after transformation using a modification of the CTAB method (Amato et al., 2007); RNA was extracted 51 days after transformation by Trizol (Invitrogen) and purified by PureLink RNA minikit (Invitrogen), then reverse transcribed by SuperScript III kit (Invitrogen) to obtain cDNAs; proteins were extracted 55 days after transformation with the following protocol:
- Collect cells by centrifugation (3000 rpm 15 minutes)
- Discard the supernatants and resuspend pellets into lysis buffer (Tris HCl 50 mM, SDS 2%. pH 6.8)
- Vortex to destroy cell membranes and leave at room temperature for 30 minutes
- Centrifuge, collect the supernatant and quantify proteins by BCA protocol (Pierce).

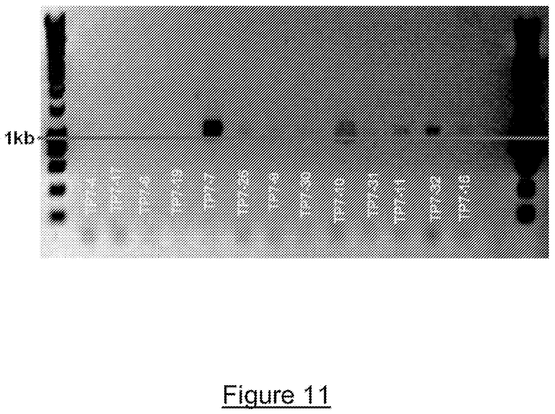

1.6 µg total RNA was reverse transcribed as described before and 1 µl of the cDNAs was amplified using meganuclease-specific primers in order to verify TP7 ORF transcription (figure 11). One out of the 13 cultures showed a strong band at around 1 kb (the expected size for TP7 single chain meganuclease). For the other strains, much weaker bands were recorded, but still a relative level of TP7 mRNA was detected.

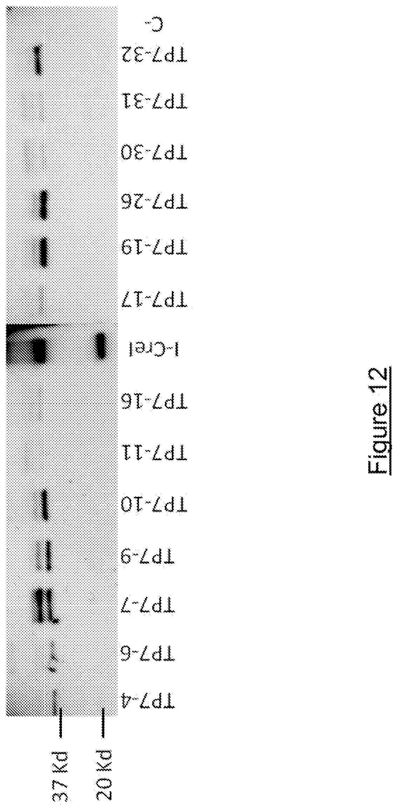

30 µg total protein extracts were loaded on polyacrylamide gel for electrophoretic separation and subsequent western blot analyses (figure 12). After electrophoresis, proteins were transferred to nitrocellulose membranes and hybridized with a rabbit polyclonal anti-I-Crel N75 (1:20000) that recognizes all engineered meganucleases (Cellectis SA). Revelation was made using a goat anti-rabbit IgG horseradish peroxidase conjugated secondary antibody (1:5000). Incubation with chemo luminescent Luminol Reagent produces light that is detected on a photographic film.

### 4) Deep sequencing analysis

100 ng genomic DNA of each culture were amplified by PCR using primers listed in Table 1 below. The primer pairs were designed to amplify a region surrounding the TP7 target. 370 bp amplicons were produced containing specific sequences to be bound onto magnetic beads for deep sequencing and specific recognition regions to univocally label each amplicon. Non homologous end-joining (NHEJ) events produced by the meganuclease TP7 were estimated by deep sequencing. TOT sequences were automatically analysed

**Table 1: 14 forward primers and one reverse primer for deep sequencing of a 370 bp amplicon spanning over the TP7 target. Each forward primer contains a MID (Multipled Identifier) region required to univocally recognize the amplicon (marked boldface in table), an adapter to bind the amplicons to magnetic beads (marked plainface in table) and the region-specific sequence (marked italics in table) to prime DNA polymerase replication.**

| name | sequence 5'-3' | SEQ ID NO : |
|---|---|---|
| MID-129-TP7-4Fw | CCATCTCATCCCTGCGTGTCTCCGACTCAG**CAGACGTCTG***GACGCAGCATTTAGCCATGAAGGT* | 74 |
| MID-130-TP7-6Fw | CCATCTCATCCCTGCGTGTCTCCGACTCAG**CAGTACTGCG***GACGCAGCATTTAGCCATGAAGGT* | 75 |
| MID-131-TP7-7Fw | CCATCTCATCCCTGCGTGTCTCCGACTCAG**CGACAGCGAG***GACGCAGCATTTAGCCATGAAGGT* | 76 |
| MID-132-TP7-9Fw | CCATCTCATCCCTGCGTGTCTCCGACTCAG**CGATCTGTCG***GACGCAGCATTTAGCCATGAAGGT* | 77 |
| MID-133-TP7-10Fw | CCATCTCATCCCTGCGTGTCTCCGACTCAG**CGCGTGCTAG***GACGCAGCATTTAGCCATGAAGGT* | 78 |
| MID-134-TP7-11Fw | CCATCTCATCCCTGCGTGTCTCCGACTCAG**CGCTCGAGTG***GACGCAGCATTTAGCCATGAAGGT* | 79 |
| MID-135-TP7-16Fw | CCATCTCATCCCTGCGTGTCTCCGACTCAG**CGTGATGACG***GACGCAGCATTTAGCCATGAAGGT* | 80 |
| MID-136-TP7-17Fw | CCATCTCATCCCTGCGTGTCTCCGACTCAG**CTATGTACAG***GACGCAGCATTTAGCCATGAAGGT* | 81 |
| MID-137-TP7-19Fw | CCATCTCATCCCTGCGTGTCTCCGACTCAG**CTCGATATAG***GACGCAGCATTTAGCCATGAAGGT* | 82 |
| MID-138-TP7-26Fw | CCATCTCATCCCTGCGTGTCTCCGACTCAG**CTCGCACGCG***GACGCAGCATTTAGCCATGAAGGT* | 83 |
| MID-139-TP7-30Fw | CCATCTCATCCCTGCGTGTCTCCGACTCAG**CTGCGTCACG***GACGCAGCATTTAGCCATGAAGGT* | 84 |
| MID-140-TP7-31Fw | CCATCTCATCCCTGCGTGTCTCCGACTCAG**CTGTGCGTCG***GACGCAGCATTTAGCCATGAAGGT* | 85 |
| MID-141-TP7-32Fw | CCATCTCATCCCTGCGTGTCTCCGACTCAG**TAGCATACTG***GACGCAGCATTTAGCCATGAAGGT* | 86 |
| MID-142-TP7-43Fw | CCATCTCATCCCTGCGTGTCTCCGACTCAG**TATACATGTG***GACGCAGCATTTAGCCATGAAGGT* | 87 |
| MID-143-TP7-wtFw | CCATCTCATCCCTGCGTGTCTCCGACTCAG**TATCACTCAG***GACGCAGCATTTAGCCATGAAGGT* | 88 |
| DeepTP7Rv | CCTATCCCCTGTGTGCCTTGGCAGTCTCAG*AGCTCACGCGGGCTCGTCAT* | 89 |

### 5) Knock-out by targeted knock-in

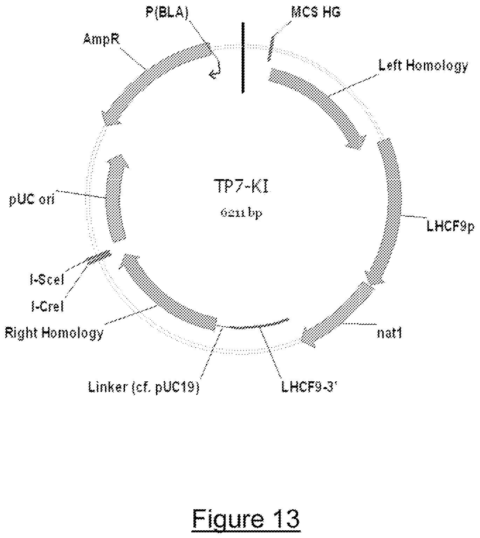

Using the meganuclease TP7 that hits on chromosome 3 into the gene encoding for the protein ID: 261853 a knock-in is produced by co-transfecting the TP7 expression plasmid (figure 8, SEQ ID NO: 71) and the knock-in matrix (figure 13, SEQ ID NO: 73). The latter plasmid is constructed by cloning two 920 bp-regions flanking the TP7 target (SEQ ID NO: 73), about 400 bp up- and downstream this locus. The left homology (located upstream the TP7 target) is amplified by PCR from the genome of the diatom *T. pseudonana* using the forward primer NotI-TP7LH-Fw (SEQ ID NO: 90, Table 2 below) and the reverse primer PstI-TP7LH-Rv (SEQ ID NO: 91, Table 2 below). The former primer introduced a NotI restriction site in 5' while the latter a PstI restriction site in 3'. This region is cloned by digestion-ligation into the nat plasmid (figure 9, SEQ ID NO: 72) upstream the *nat*l gene expression cassette giving rise to the TP7-LH plasmid. Following the same strategy, the right homology (located downstream the TP7 target) is amplified by PCR from the *T. pseudonana* genome using the primers EcoRI-TP7RH-Fw (SEQ ID NO: 92) and AflII-TP7RH-Rv (SEQ ID NO: 93). EcoRI and AflII are the 5' and 3' restriction sites carried by the forward and reverse primer respectively. The TP7-LH plasmid is digested by MfeI and AflII enzymes, the PCR product by EcoRI and AflII (note that EcoRI and MfeI are compatible enzymes, both leaving an AATT sticky end) then ligated to produce the TP7-KI matrix (figure 13, SEQ ID NO: 73).

**Table 2: list of primers used for cloning of left and right homologies in the knock-in matrix.**

| name | notes | organism or plasmid | sequence 5'-3' (restriction enzyme site in bold) |
|---|---|---|---|
| Noti-TP7LH-Fw | carries a **NotI** site in 5' | *Thalassiosira pseudonana* | atat**gcggccgc**caagcttcatttgttggccg (SEQ ID NO : 90) |
| PstI-TP7LH-Rv | Cerries a **PstI** site in 5' | *Thalassiosira pseudonana* | ttaa**ctgcag**tgacgagcccccgtgagctg (SEQ ID NO : 91) |
| EcoRI-TP7RH-Fw | Carries a **EcoRI** site in 5' to be cloned in the Mfel-AflII fragment of the nat plasmid | *Thalassiosira pseudonana* | atat**gaattc**tcgcttggagctatcattac (SEQ ID NO : 92) |
| AflII-TP7RH-Rv | carries a **AflII** site in 5' | *Thalassiosira pseudonana* | ttaa**cttaag**atgagaacaggtgaattggcgg (SEQ ID NO : 93) |

10⁷ cells are co-electroporated following the protocol described above in paragraph 2) with the two plasmids nat (SEQ ID NO: 72) and TP7-KI (SEQ ID NO: 73). The day after electroporation, cells are plated onto selective plates (100 µg nourseothricin). Nourseothricin-positive colonies are screened by PCR in order to check whether the *nat*1 gene is randomly integrated or is integrated between the left and right homology into the gene.

### LIST OF REFERENCES CITED IN THE DESCRIPTION

Norton, T. A., Melkonian, M., & Andersen, R. A. (1996) Phycologia 35, 308-326
Steinbrenner, J. & Sandmann, G. (2006) Appl. Environ. Microbiol. 72, 7477-7484
Mogedas, B., Casal, C., Forjan, E., & Vilchez, C. (2009) J Biosci Bioeng 108, 47-51.
Palmer, J. D., Soltis, D. E., & Chase, M. W. (2004) Am. J. Bot. 91, 1437-1445
Tyler, B. M., Tripathy, S., Zhang, X., Dehal, P., Jiang, R. H. Y., Aerts, A., Arredondo, F. D., Baxter, L., Bensasson, D., Beynon, J. L. et al. (2006) Science 313, 1261-1266
Armbrust, E. V., Berges, J. A., Bowler, C., Green, B. R., Martinez, D., Putnam, N. H., Zhou, S., Allen, A. E., Apt, K. E., Bechner, M. et al. (2004) Science 306, 79-86.
Bowler, C., Allen, A. E., Badger, J. H., Grimwood, J., Jabbari, K., Kuo, A., Maheswari, U., Martens, C., Maumus, F., Otillar, R. P. et al. (2008) Nature. 456, 239-244.
Bowler, C., Allen, A. E., Badger, J. H., Grimwood, J., Jabbari, K., Kuo, A., Maheswari, U., Martens, C., Maumus, F., Otillar, R. P. et al. (2008) Nature. 456, 239-244.
Lusic et al Advanced Functional Materials 2006
Rouet et al. PNAS 1994 91:6064-6068
Rouet et al. Mol Cell Biol. 1994 14 :8096-8106
Choulika et al. Mol Cell Biol. 1995 15 :1968-1973
Puchta et al. PNAS 1996 93 :5055-5060
Paques et al. Curr Gen Ther. 2007 7:49-66
Arnould et al. J Mol Biol. 2007 371:49-65
Grizot et al. NAR 2009 37:5405
Galetto et al. Expert Opin Biol Ther. 2009 9:1289-303
Norton, T. A., Melkonian, M., & Andersen, R. A. (1996) Phycologia 35, 308-326
Steinbrenner, J. & Sandmann, G. (2006) Appl. Environ. Microbiol. 72, 7477-7484
Mogedas, B., Casal, C., Forjan, E., & Vilchez, C. (2009) J Biosci Bioeng 108, 47-51.
Palmer, J. D., Soltis, D. E., & Chase, M. W. (2004) Am. J. Bot. 91, 1437-1445
Tyler, B. M., Tripathy, S., Zhang, X., Dehal, P., Jiang, R. H. Y., Aerts, A., Arredondo, F. D., Baxter, L., Bensasson, D., Beynon, J. L. et al. (2006) Science 313, 1261-1266
Armbrust, E. V., Berges, J. A., Bowler, C., Green, B. R., Martinez, D., Putnam, N. H., Zhou, S., Allen, A. E., Apt, K. E., Bechner, M. et al. (2004) Science 306, 79-86.
Bowler, C., Allen, A. E., Badger, J. H., Grimwood, J., Jabbari, K., Kuo, A., Maheswari, U., Martens, C., Maumus, F., Otillar, R. P. et al. (2008) Nature. 456, 239-244.
Bowler, C., Allen, A. E., Badger, J. H., Grimwood, J., Jabbari, K., Kuo, A., Maheswari, U., Martens, C., Maumus, F., Otillar, R. P. et al. (2008) Nature. 456, 239-244.
Lusic et al Advanced Functional Materials 2006
Rouet et al. PNAS 1994 91:6064-6068
Rouet et al. Mol Cell Biol. 1994 14 :8096-8106
Choulika et al. Mol Cell Biol. 1995 15 :1968-1973
Puchta et al. PNAS 1996 93 :5055-5060
Paques et al. Curr Gen Ther. 2007 7:49-66
Arnould et al. J Mol Biol. 2007 371:49-65
Grizot et al. NAR 2009 37:5405
Galetto et al. Expert Opin Biol Ther. 2009 9:1289-303
Paques et al. Curr Gen Ther. 2007 7:49-66
Arimondo et al. Mol Cell Biol. 2006 26:324-333; Simon et al. NAR 2008 36:3531-3538
Porteus et al. Nat Biotechnol. 2005 23:967-973
Cannata et al. PNAS 2008 105:9576-9581
Lee et al., 2002, EMBO J. 21: 4663-4670
Lee et al, 2003, Nature 425:415-419
Yi et al, 2003, Genes and Development 17:3011-3016
Hutvagner et al, 2001, Science 293:834-838
Bartel et al, 2004, Cell 116: 281-297
Bouvier-Nave et al. 2000, European J.of Biochem., Vol.267, pp.85-96.
Cheng et al. 2006, Gene. Vol.371, pp.112-120.
Desbois, et al. 2009, Mar Biotechnol. (NY), Vol. 11, pp.45-52.
Dunahay et al. 1996, Appl. Biochem. Biotechnol., Vol.57, pp.223-231.
Frommolt et al. 2008, Mol. Biol. Evol., Vol.25, pp.2653-2667.
Gordon et al. 2008, Trends in Biotechnology, Vol.27, pp.116-127.
Hentzer and Givskov. 2003, J. Clin Invest., Vol.112, pp.1300-1307.
Jako et al. 2001, Plant Physiology, Vol.126, pp.861-874.
Junchao et al. 2008, J. of Phycology, Vol.44, pp.684-690.
Kuehl et al. 2009, Antimicrobial Agents and Chemotherapy, Vol.53, pp.4159-4166.
Li et al. 2010, Metabolic Engineering,, Vol.12, pp.387-391.
Li et al. 2010, Biotechnology and Bioengineering. Pub online May 20 (Epub ahead of print).
Marillia et al. 2003, J. of Exp. Botany,Vol.54, pp.259-270
Roesler et al. 1997, Plant Physiology, Vol.113, pp.75-81.
Teplitski et al. 2004, Plant Physiology, Vol.134, pp.137-146.
Thamatrakoln and Hildebrand. 2008, Plant Physiology, Vol.146, pp.1397-1407.
Vigeolas et al. 2007, Plant Biotech. J., Vol.5, pp.431-441.
Weselake et al. 2008, J. of Exp. Botany, Vol.59, pp.3543-3549.
Williams P. 2007, Microbiology, Vol.153, pp.3923-3938.
Zabawinski et al. 2001, J. of Bacteriology, Vol.183, pp.1069-1077.
Zheng et al. 2008, Nature Genetics, Vol.40, pp.367-372.
Zou et al. 1997, Plant Cell, Vol.9, pp.909-923.
Amato et al. 2007, Protist 158:193-207.

## Claims

1. A method for targeted genomic engineering in an algal cell comprising introducing at least an endonuclease variant in said algal cell to induce a double-stranded cleavage at a site of interest into the nuclear genome of said algal cell,
wherein said endonuclease variant is obtained by genetic engineering or by random mutation from a wild-type endonuclease, and recognizes and specifically cleaves a target sequence that is different from the target sequence of the corresponding wild-type endonuclease and
wherein said algal cell is a diatom.

2. The method according to claim 1, wherein said endonuclease variant is introduced into algal cells by introduction of DNA into the algal nuclei.

3. The method according to claim 2, wherein the DNA is introduced by a method selected from the group consisting of: electroporation and particle inflow gun bombardment methods.

4. The method of claim 1 for obtaining a knocked-out alga comprising:
a) Providing an endonuclease variant capable of inducing a double-stranded cleavage at a site of interest into the nuclear genome of an algal cell;
b) Introducing said endonuclease into an algal cell;
c) Isolating a modified algal cell knocked-out for the gene located at the site of interest,
wherein said alga is a diatom.

5. The method according to any one of claims 1 to 4, wherein a targeted knock-out in alga is induced by said endonuclease at the site of interest in said genome.

6. The method according to any one of claims 1 to 5, wherein at least one knock-in template is inserted at the genomic site of interest by further introducing into the algal cell of a knock-in template wherein said template is flanked by sequences sharing homologies with the region surrounding the genomic DNA cleavage site of interest.

7. The method according to claim 6 wherein said template comprises a nucleic acid encoding a positive selectable marker.

8. The method of claim 7 wherein said selectable marker is N-acetyltransferase 1 (Nat1) conferring resistance to Nourseothricin.

9. The method according to any one of claims 6 to 8, to insert a transgene into the nuclear genome of the algae without modifying the expression of the two nearest genes located centromeric and telomeric to the site of interest.

10. The method according to any one of claims 6 to 9 wherein said template comprises multiple transgenes.

11. The method according to any one of claims 1 to 10 wherein said endonuclease is a meganuclease.

12. The method of claim 11 wherein said meganuclease is an engineered I-Crel, selected from the group consisting of a homodimeric, heterodimeric, obligate heterodimer and single chain variant.

13. The method according to any one of claims 1 to 10 wherein said endonuclease is a chimeric Zinc-Finger nuclease resulting from the fusion of engineered zinc-finger domains with the catalytic domain of a restriction enzyme.

14. The method according to anyone of claims 1 to 13 wherein algae are selected from the group consisting of *Amphora, Chaetoceros, Cyclotella, Cylindrotheca, Monoraphidium, Navicula, Nitzschia, Phaeodactylum and Thalassiosira.*

## Patentansprüche

1. Verfahren zur gezielten Veränderung des Genoms in einer Algenzelle, welches das Einführen mindestens einer Endonuklease-Variante in die Algenzelle umfasst, um an einer interessierenden Stelle einen Doppelstrangbruch im Kerngenom der Algenzelle zu induzieren,
wobei die Endonuklease-Variante durch gentechnische Veränderung oder durch zufällige Mutation aus einer Wildtyp-Endonuklease erhalten ist und eine Zielsequenz erkennt und spezifisch schneidet, welche von der Zielsequenz der entsprechenden Wildtyp-Endonuklease verschieden ist und wobei die Alge eine Kieselalge ist.

2. Verfahren nach Anspruch 1, wobei die Endonuklease-Variante durch Einführung von DNA in die Algenzellkerne in Algenzellen eingeführt wird.

3. Verfahren nach Anspruch 2, wobei die DNA durch ein Verfahren eingeführt wird, welches ausgewählt ist aus Elektroporation und Particle-Inflow-Gun-Beschussverfahren.

4. Verfahren nach Anspruch 1 zum Erhalten einer Knock-out-Alge, welches umfasst:
a) Bereitstellen einer Endonuklease-Variante, die geeignet ist, an einer interessierenden Stelle einen Doppelstrangbruch im Kerngenom der Algenzelle zu induzieren;
b) Einführen der Endonuklease in eine Algenzelle;
c) Isolieren einer modifizierten Algenzelle, in welcher das an der interessierenden Stelle befindliche Gen abgeschaltet ist, wobei die Alge eine Kieselalge ist.

5. Verfahren nach einem der Ansprüche 1 bis 4, wobei durch die Endonuklease in Algen ein gezielter Knock-out an der interessierenden Stelle in dem Genom induziert wird.

6. Verfahren nach einem der Ansprüche 1 bis 5, wobei an der interessierenden Stelle im Genom zumindest ein Knock-in-Template eingefügt wird, indem in die Algenzelle zudem ein Knock-in-Template eingeführt wird, wobei das Template von Sequenzen flankiert ist, die Homologien mit der Region teilen, welche die interessierende Stelle des DNA-Schnitts im Genom umgibt.

7. Verfahren nach Anspruch 6, wobei das Template eine Nukleinsäure umfasst, die für einen positiven selektierbaren Marker kodiert.

8. Verfahren nach Anspruch 7, wobei der selektierbare Marker N-Acetyltransferase 1 (Nat1) ist, die Resistenz gegen Nourseothricin verleiht.

9. Verfahren nach einem der Ansprüche 6 bis 8 zum Einfügen eines Transgens in das Kerngenom der Algen, ohne die Expression der zwei nächstliegenden, zentromerisch und telomerisch zu der interessierenden Stelle befindlichen Gene zu verändern.

10. Verfahren nach einem der Ansprüche 6 bis 9, wobei das Template mehrere Transgene umfasst.

11. Verfahren nach einem der Ansprüche 1 bis 10, wobei die Endonuklease eine Meganuklease ist.

12. Verfahren nach Anspruch 11, wobei die Meganuklease eine veränderte I-Crel ist, welche ausgewählt ist aus der Gruppe bestehend aus einer homodimeren, heterodimeren, obligat-heterodimeren und einzelkettigen Variante.

13. Verfahren nach einem der Ansprüche 1 bis 10, wobei die Endonuklease eine chimäre Zinkfingernuklease ist, die aus der Fusion veränderter Zinkfingerdomänen mit der katalytischen Domäne eines Restriktionsenzym stammt.

14. Verfahren nach einem der Ansprüche 1 bis 13, wobei Algen ausgewählt sind aus der Gruppe bestehend aus *Amphora, Chaetoceros, Cyclotella, Cylindrotheca, Monoraphidium, Navicula, Nitzschia, Phaeodactylum* und *Thalassiosira.*

## Revendications

1. Procédé de génie génomique ciblé dans une cellule d'algue, comprenant l'introduction d'au moins un variant d'endonucléase dans ladite cellule d'algue afin d'induire un clivage double brin au niveau d'un site d'intérêt dans le génome nucléaire de ladite cellule d'algue,
dans lequel ledit variant d'endonucléase est obtenu par génie génétique ou par une mutation aléatoire d'une endonucléase de type sauvage, et reconnaît et clive spécifiquement une séquence cible qui est différente de la séquence cible de l'endonucléase de type sauvage correspondante, et dans lequel ladite cellule d'algue est une diatomée.

2. Procédé selon la revendication 1, dans lequel ledit variant d'endonucléase est introduit dans les cellules d'algue par l'introduction d'ADN dans les noyaux des algues.

3. Procédé selon la revendication 2, dans lequel l'ADN est introduit par un procédé sélectionné dans le groupe consistant en : des procédés d'électroporation et de bombardement par canon à particules.

4. Procédé selon la revendication 1 pour obtenir une algue comportant une invalidation génique, comprenant :
a) la mise à disposition d'un variant d'endonucléase capable d'induire un clivage double brin au niveau d'un site d'intérêt dans le génome nucléaire d'une cellule d'algue ;
b) l'introduction de ladite endonucléase dans une cellule d'algue ;
c) l'isolement d'une cellule d'algue modifiée comportant une invalidation génique du gène situé au niveau du site d'intérêt, où ladite algue est une diatomée.

5. Procédé selon l'une quelconque des revendications 1 à 4, dans lequel une invalidation génique ciblée dans l'algue est induite par ladite endonucléase au niveau du site d'intérêt dans ledit génome.

6. Procédé selon l'une quelconque des revendications 1 à 5, dans lequel au moins une matrice de remplacement génique est insérée au niveau du site génomique d'intérêt en introduisant en outre, dans la cellule d'algue, une matrice de remplacement génique, où ladite matrice est flanquée par des séquences partageant des homologies avec la région entourant le site de clivage d'ADN génomique d'intérêt.

7. Procédé selon la revendication 6, dans lequel ladite matrice comprend un acide nucléique codant pour un marqueur de sélection positive.

8. Procédé selon la revendication 7, dans lequel ledit marqueur de sélection est la N-acétyltransférase 1 (Nat1) conférant une résistance à la nourséothricine.

9. Procédé selon l'une quelconque des revendications 6 à 8, pour insérer un transgène dans le génome nucléaire des algues sans modifier l'expression des deux gènes les plus proches situés en position centromérique et télomérique par rapport au site d'intérêt.

10. Procédé selon l'une quelconque des revendications 6 à 9, dans lequel ladite matrice comprend de multiples transgènes.

11. Procédé selon l'une quelconque des revendications 1 à 10, dans lequel ladite endonucléase est une méganucléase.

12. Procédé selon la revendication 11, dans lequel ladite méganucléase est une I-CreI modifiée, sélectionnée dans le groupe consistant en un variant homodimérique, hétérodimérique, à hétérodimère obligatoire et à chaîne unique.

13. Procédé selon l'une quelconque des revendications 1 à 10, dans lequel ladite endonucléase est une nucléase à doigts de zinc chimérique résultant de la fusion de domaines à doigts de zinc modifiés avec le domaine catalytique d'une enzyme de restriction.

14. Procédé selon l'une quelconque des revendications 1 à 13, dans lequel les algues sont sélectionnées dans le groupe consistant en *Amphora, Chaetoceros, Cyclotella, Cylindrotheca, Monoraphidium, Navicula, Nitzschia, Phaeodactylum* et *Thalassiosira.*
